# EUROPEAN PATENT APPLICATION

(11) **EP 4 681 754 A1**
(43) Date of publication of application: **21.01.2026**
(21) Application number: 25182160.9
(22) Date of filing: 11.06.2025
(51) Int. Cl.: A61M 15/08

(54) **GAS DELIVERY APPARATUS FOR NASAL APPLICATION**

(30) Priority: 25.06.2024 US 202418753277; 25.03.2025 US 202519089927
(71) Applicant: James L. Orrington, II D.D.S., P.C., Chicago, IL 60619 (US)
(72) Inventor: ORRINGTON, II James L., Flossmoor, 60442 (US); ORRINGTON-MYERS, Janie, Terre Haute, 47803 (US); HENRY, Brandi Marie, Crystal Lake, 60012 (US)
(74) Representative: South, Nicholas Geoffrey

(57) **Abstract**

A gas delivery apparatus for nasal application is provided for purifying and/or reducing undesirable materials from air and/or gas intended to enter a nasal cavity. The gas delivery apparatus for nasal application may include a housing component, power component, fan component, filtration component, cap, germicidal irradiation component, aroma component, nose plug component, monitoring component, and medicinal component.

## Description

### CROSS-REFERENCE TO RELATED APPLICATIONS

This application claims priority from U.S. patent application serial number 18/753,277 (the '277 application) filed on June 25, 2024, and from U.S. patent application serial number 19/089,927 filed March 25, 2025, which is a continuation of the '277 application. The '277 application is a continuation-in-part of PCT application serial number PCT/US22/52085, filed on December 7, 2022. The '277 application is a continuation-in-part of U.S. patent application serial numbers 29/934,132, filed on March 23, 2024, now patent number 01064286; 17/994,293, filed on November 26, 2022; 17/982,355, filed on November 7, 2022; and 29/888,886, filed on April 5, 2023, now patent number D1024333. The foregoing applications and patent are incorporated in their entirety herein by reference.

### FIELD OF THE INVENTION

The present disclosure relates to a gas delivery apparatus for nasal application. More particularly, the disclosure relates to purifying and/or reducing undesirable materials from gas intended to enter a nasal cavity.

### BACKGROUND

For generations, and most particularly since the onset of the COVID-19 pandemic, societies have been cognizant of environmental conditions that may present health hazards. Today, vigilance in the face of air-borne viruses and other hazardous agents is prevalent throughout the world. Devices that help prevent inhalation of viruses and other harmful agents in the environment are in high demand by those seeking to maintain optimal health and not contract COVID-19 or other harmful airborne viruses and agents.

Carbon air filters are well-known to be effective in removing hazardous gaseous chemicals, as well as certain odors, from the air. HEPA (high efficiency particulate absorbing) air filters, by contrast, are mechanical in nature. HEPA filters are well-known to remove undesired particulate matter from the air. Those of skill in the art readily appreciate that carbon air filters and HEPA filters may be used together.

Ultraviolet (UV) light consists of electromagnetic radiation with wavelengths shorter than visible light but longer than x-rays. UV light is well-known to exhibit anti-viral and anti-bacterial properties. Short wavelength UV light (UV-C) is considered "germicidal UV." Nucleic acids that make up DNA of viruses and bacteria may exhibit defects when exposed to UV-C light. These defects may prevent effective replication, thus eliminating or substantially reducing the ability of viruses and bacteria to replicate.

The sense of smell plays a significant role in the physiological effects of mood, stress, and working capacity. A large family of olfactory receptors present in humans are genetically designed to detect thousands of different fragrances. The positive impact of prolonged exposure to certain types of aromas and aromas on psychophysiological activities of humans has been recognized for years. See, e.g., K. Sowndhararajan & S. Kim, Influence of Fragrances on Human Psychophysiologylogical Activity With Special Reference to Human Electroencephalographic Response, 84(4) Sci. Pharm. 724 (2016). Therefore, a need exists to solve the deficiencies present in the prior art.

What is needed is an apparatus to remove contaminants, particulates, and other undesirable elements from air prior to inhalation by a user. What is needed is an air purification apparatus to reduce contact of undesirable elements with tissue within a nasal cavity or otherwise related to the respiratory system. What is needed is a device to remove impurities from air prior to entering the nasal cavity by performing filtration and/or germicidal irradiation. What is needed is a portable device to be comfortably inserted into a nostril and perform air purification functions. What is needed is an air and gas purification device that can detect a condition relating to incoming and outgoing air and at least partially purify same.

### SUMMARY

An aspect of the disclosure advantageously provides an apparatus to remove contaminants, particulates, and other undesirable elements from air prior to inhalation by a user. An aspect of the disclosure advantageously provides an air purification apparatus to reduce contact of undesirable elements with tissue within a nasal cavity or otherwise related to the respiratory system. An aspect of the disclosure advantageously provides a device to remove impurities from air prior to entering the nasal cavity by performing filtration and/or germicidal irradiation. An aspect of the disclosure advantageously provides a portable device to be comfortably inserted into a nostril and perform air purification functions. An aspect of the disclosure advantageously provides an air and gas purification device that can detect a condition relating to incoming and outgoing air and at least partially purify same.

Accordingly, the disclosure may feature a gas purification apparatus for nasal application including a housing, a gas treatment component, a nose plug component, and a cap. The housing may substantially enclose an interior space. The gas treatment component may be located within the interior space to at least partially purify gas for delivery to a nasal cavity. The gas treatment component may comprise a filtration component to remove impurities from the gas. The nose plug component may operatively be attached to a nose end of the housing, which may be removably inserted into a nostril to form an at least partial seal with a nasal cavity. The gas that may be passed through the filtration component may be directed to the nasal cavity through the nose plug component. The cap may be removably attached to a cap end of the housing to receive the gas delivered from a gas source via a delivery line. The cap end may be distal to the nose end.

In another aspect, the delivery line may have a delivery line diameter which may be selectively installed to the cap such that the gas from the delivery line may be received through a gas receiving aperture of the cap.

In another aspect, the delivery line may be received by the cap having a filtration diameter. The filtration diameter may be larger than the delivery line diameter which may increase a filtration efficacy of the filtration component.

In another aspect, the cap may comprise a cannula to receive the delivery line through which gas may be flowed. The delivery line diameter may be larger than a cannula diameter of the cannula.

In another aspect, the impurities may comprise microorganisms.

In another aspect, the gas source may be a continuous positive airway pressure machine.

In another aspect, the gas source may be a pressurized tank.

In another aspect, the gas treatment component may further comprise a fan component and a power component. The fan component may actively pass the gas through the filtration component. The power component may be located substantially within the interior space to provide electrical power to the gas treatment component.

In another aspect, the housing may have a cap receiving feature. The cap receiving feature may be at least partially flexible to allow for installation and removal of the cap.

In another aspect, the apparatus may further comprise an auxiliary aperture on the housing.

In another aspect, the apparatus may further comprise a drying agent to at least partially remove moisture within the interior space of the housing. The at least part of the moisture may vent through the auxiliary aperture.

In another aspect, the gas treatment component may further comprise an aroma component which may comprise an aroma reservoir to reversibly hold an aromatic material which may have an aroma. The aroma may at least be partially transferred from the aromatic material into the gas.

In another aspect, the gas treatment component may further comprise a medicinal component which may comprise a medicinal reservoir to reversibly hold a medicine. The medicine may at least be partially transferred from the medicinal component into the gas.

In another aspect, the filtration component may be replaceable and may comprise a high efficiency particulate absorbing (HEPA) filter.

In another aspect, the apparatus may further comprise a monitoring component which may indicate a likelihood of microorganisms being present in an environmental space accessible via the nose plug component.

According to an alternative embodiment, the disclosure may feature a gas purification apparatus for nasal application that comprises a housing, a gas treatment component, a nose plug component, and a cannula of a cap. The housing may substantially enclose an interior space. The gas treatment component may be located within the interior space to at least partially purify gas and at least partially remove moisture from the gas for delivery to a nasal cavity. The gas treatment component may further comprise a high efficiency particulate absorbing (HEPA) filter to remove impurities from the gas. The nose plug component may be operatively attached to a nose end of the housing to be removably inserted into a nostril to form an at least partial seal with a nasal cavity. The seal may allow gas passed through the filtration component to be directed into the nasal cavity through the nose plug component. The cannula of a cap may be removably attached to a cap end of the housing to receive the gas delivered from a gas source via a delivery line. The cap end may be distal to the nose end. The delivery may have a delivery line diameter which may be selectively installed to the cannula such that the gas from the delivery line is received through a gas receiving aperture of the cannula. The delivery line may be received by the cap which may have a filtration diameter that is larger than the delivery line diameter which may increase a filtration efficacy of the high efficiency particulate absorbing (HEPA) filter.

In another aspect, the gas treatment component may further comprise a medicinal component which may comprise a medicinal reservoir to reversibly hold a medicine. The medicine may at least be partially transferred from the medicinal component into the gas.

In another aspect, the apparatus may further comprise a monitoring component which may indicate a likelihood of microorganisms being present in an environmental space accessible via the housing.

In another aspect, the housing may have a cap receiving feature. The cap receiving feature may be at least partially flexible to allow for installation and removal of the cap.

In another aspect, the apparatus may further comprise an auxiliary aperture on the housing.

Terms and expressions used throughout this disclosure are to be interpreted broadly. Terms are intended to be understood respective to the definitions provided by this specification. Technical dictionaries and common meanings understood within the applicable art are intended to supplement these definitions. In instances where no suitable definition can be determined from the specification or technical dictionaries, such terms should be understood according to their plain and common meaning. However, any definitions provided by the specification will govern above all other sources.

Various objects, features, aspects, and advantages described by this disclosure will become more apparent from the following detailed description, along with the accompanying drawings in which like numerals represent like components.

### BRIEF DESCRIPTION OF THE DRAWINGS

FIG. 1 is a perspective view of an illustrative air purification apparatus, according to an embodiment of this disclosure.
FIG. 2 is a perspective view of an illustrative air purification apparatus shown at an alternate angle with the housing installed, according to an embodiment of this disclosure.
FIG. 3 is a front elevation view of an illustrative air purification apparatus, according to an embodiment of this disclosure.
FIG. 4 is a rear elevation view of an illustrative air purification apparatus, according to an embodiment of this disclosure.
FIG. 5 is a bottom plan view of an illustrative air purification apparatus, according to an embodiment of this disclosure.
FIG. 6 is a top plan view of an illustrative air purification apparatus, according to an embodiment of this disclosure.
FIG. 7 is a cross-sectional view of an illustrative air purification apparatus shown in FIG. 3, according to an embodiment of this disclosure.
FIG. 8 is an exploded perspective view of an illustrative air purification apparatus, according to an embodiment of this disclosure.
FIG. 9 is a perspective view of an illustrative air purification apparatus included by a case aspect, according to an embodiment of this disclosure.
FIG. 10 is a partially exploded perspective view of an illustrative air purification apparatus included by a case aspect, according to an embodiment of this disclosure.
FIG. 11 is a perspective view of an alternative embodiment of an illustrative air purification apparatus, according to an embodiment of this disclosure.
FIG. 12 is a perspective view of an alternative embodiment of an illustrative air purification apparatus showing a bottom portion, according to an embodiment of this disclosure.
FIG. 13 is a front elevation view of an alternative embodiment of an illustrative air purification apparatus, according to an embodiment of this disclosure.
FIG. 14 is a bottom plan view of an alternative embodiment of an illustrative air purification apparatus, according to an embodiment of this disclosure.
FIG. 15 is a top plan view of an alternative embodiment of an illustrative air purification apparatus, according to an embodiment of this disclosure.
FIG. 16 is a cross-sectional view of an alternative embodiment of an illustrative air purification apparatus shown in FIG. 13, according to an embodiment of this disclosure.
FIG. 17 is an exploded perspective view of an alternative embodiment of an illustrative air purification apparatus, according to an embodiment of this disclosure.
FIG. 18 is a partially exploded perspective view of an alternative embodiment of an illustrative air purification apparatus showing a replaceable filter, according to an embodiment of this disclosure.
FIG. 19 is a front perspective view of a third embodiment of an illustrative air purification apparatus, according to an embodiment of this disclosure.
FIG. 20 is a front elevation view of a third embodiment of an illustrative air purification apparatus, according to an embodiment of this disclosure.
FIG. 21 is a top plan view of a third embodiment of an illustrative air purification apparatus, according to an embodiment of this disclosure.
FIG. 22 is an exploded view of a third embodiment of an illustrative air purification apparatus, according to an embodiment of this disclosure.
FIG. 23 is an exploded view of an alternative configuration of the embodiment shown in FIG. 22, according to an embodiment of this disclosure.
FIG. 24 is a perspective view of an illustrative use of an air purification apparatus enabled by this disclosure, according to an embodiment of this disclosure.
FIG. 25 is a side elevation view of a fourth embodiment of an illustrative air purification apparatus enabled by this disclosure.
FIG. 26 is a top plan view of a fourth embodiment of an illustrative air purification apparatus, according to an embodiment of this disclosure.
FIG. 27 is a cross-sectional view of the illustrative air purification apparatus shown in FIG. 26, according to an embodiment of this disclosure.
FIG. 28 is a perspective view of an illustrative use of an air purification apparatus, according to an embodiment of this disclosure.

### DETAILED DESCRIPTION

The following disclosure is provided to describe various embodiments of an air and/or gas purification apparatus for nasal application. Skilled artisans will appreciate additional embodiments and uses of the present invention that extend beyond the examples of this disclosure. Terms included by any claim are to be interpreted as defined within this disclosure. Singular forms should be read to contemplate and disclose plural alternatives. Similarly, plural forms should be read to contemplate and disclose singular alternatives. Conjunctions should be read as inclusive except where stated otherwise.

Expressions such as "at least one of A, B, and C" should be read to permit any of A, B, or C singularly or in combination with the remaining elements. Additionally, such groups may include multiple instances of one or more element in that group, which may be included with other elements of the group. All numbers, measurements, and values are given as approximations unless expressly stated otherwise.

For the purpose of clearly describing the components and features discussed throughout this disclosure, some frequently used terms will now be defined, without limitation. The term "HEPA filter," as it is used throughout this disclosure, is defined as a high-efficiency particulate absorbing filter being, using, or containing a filter usually designed to capture a substantial portion of airborne particles, for example 99.97 percent of airborne particles measuring 0.3 micrometers or greater in diameter, from air passing through the filter. The term "ultraviolet (UV) light," as it is used throughout this disclosure, is defined as light with wavelengths shorter than that of visible light and longer than that of X-rays, including UV-C short-wavelength ultraviolet light that may be used for ultraviolet germicidal irradiation. The term "nasal," as it is used throughout this disclosure, is defined as pertaining to the nose and related features. The term "nostril," as it is used throughout this disclosure, is defined as an opening to a nasal cavity. The term "medicinal," as it is used throughout this disclosure, is defined as exhibiting therapeutic properties, including properties useful for curing disease, treating an illness or condition, and/or relieving pain.

The term "cannula," as it is used throughout this disclosure, is defined as a small variably sized tube that may assist in guiding air and/or gases. The term "microorganisms," as it is used throughout this disclosure, is defined as an organism of microscopic size, including, but not limited to, bacterium, virus, or fungi. The term "gas," as it is used throughout this disclosure, is defined as a substance that has neither independent shape nor volume and holds the property of indefinite expansion, including, but not limited to, molecules (such as oxygen, radon, and nitrogen), chemical compounds (such as nitrogen dioxide, methanal, and benzene), and mixtures of various molecules and chemical compounds (such as air, biogas, and natural gas).

Various aspects of the present disclosure will now be described in detail, without limitation. In the following disclosure, an air and/or gas purification apparatus for nasal application will be discussed. Those of skill in the art will appreciate alternative labeling of the air and/or gas purification apparatus for nasal application as a nose filter device, the air and/or gas purification apparatus for nasal application, nasal filtration device, portable powered air purification apparatus, the invention, the apparatus, or other similar names. Similarly, those of skill in the art will appreciate alternative labeling of the air and/or gas purification apparatus for nasal application as an air filtration operation, irradiation and/or filtration technique, air and/or gas quality monitoring and improvement method, method, operation, the invention, or other similar names. Skilled readers should not view the inclusion of any alternative labels as limiting in any way.

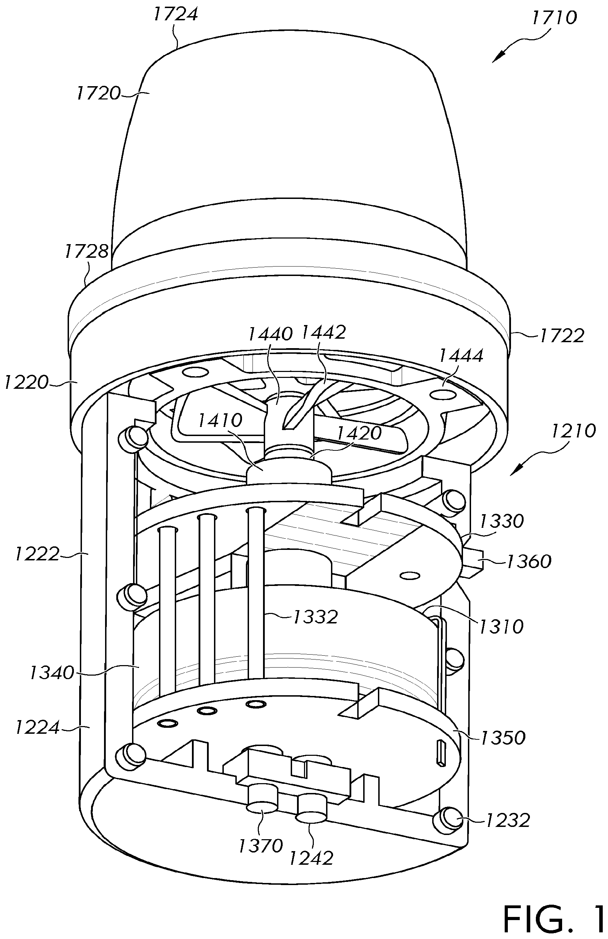

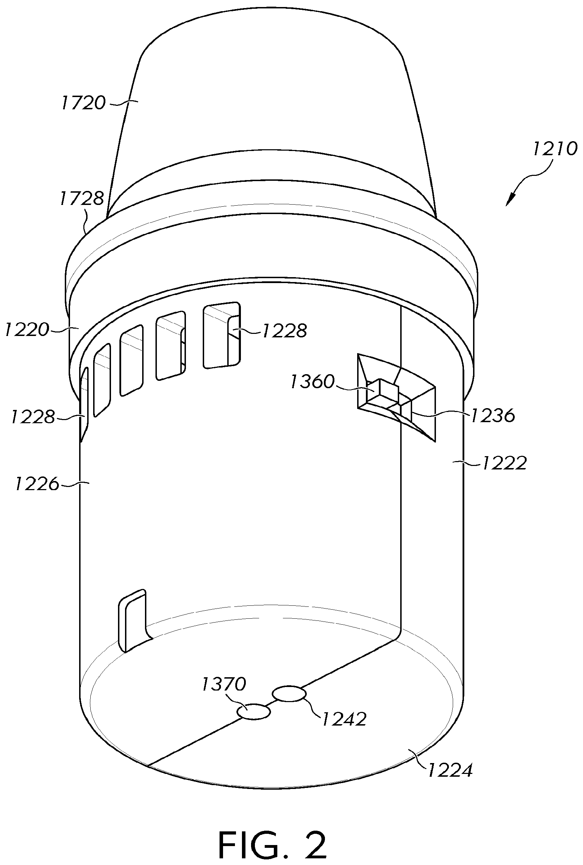

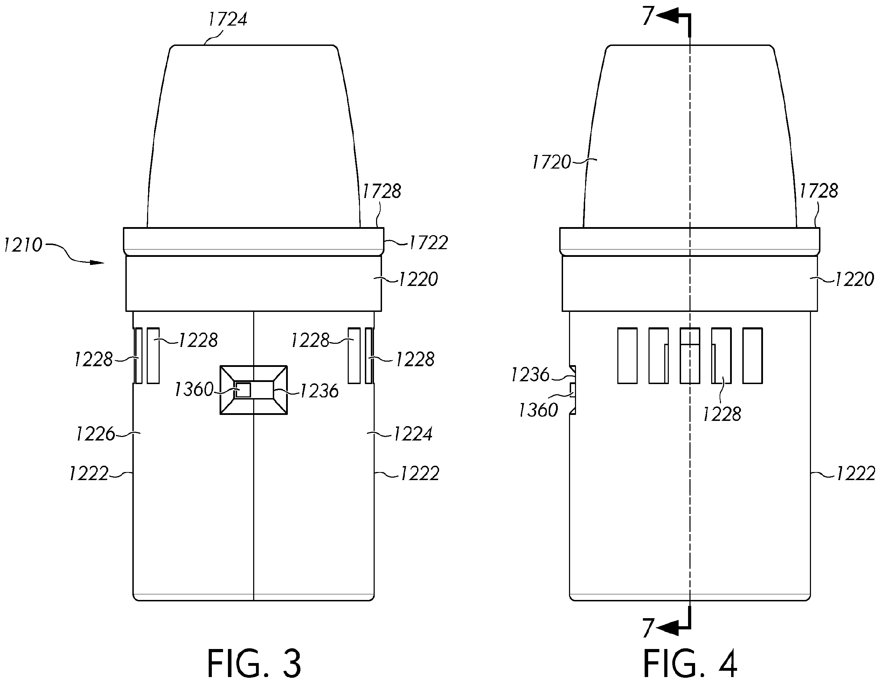

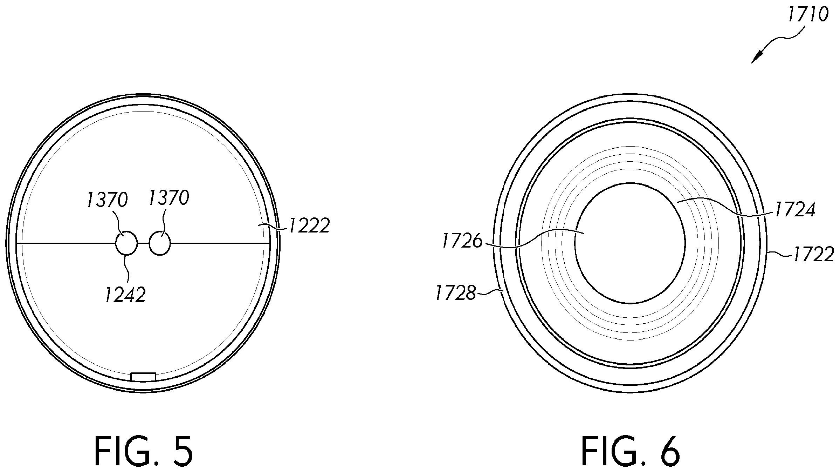

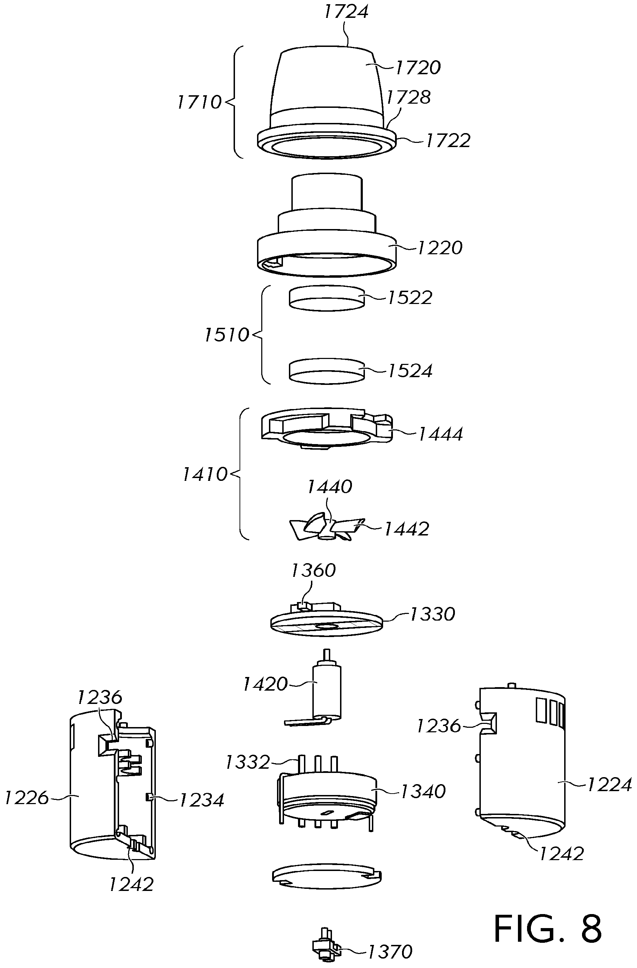

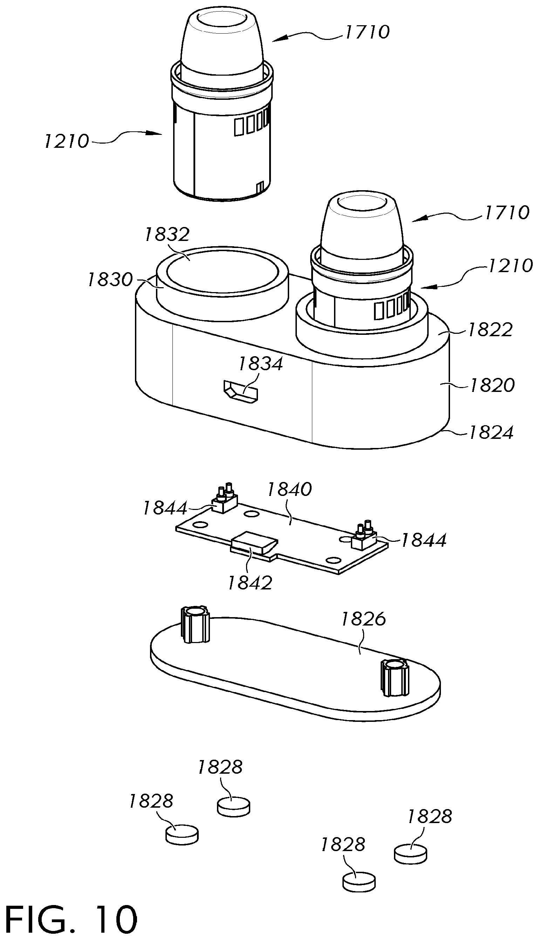

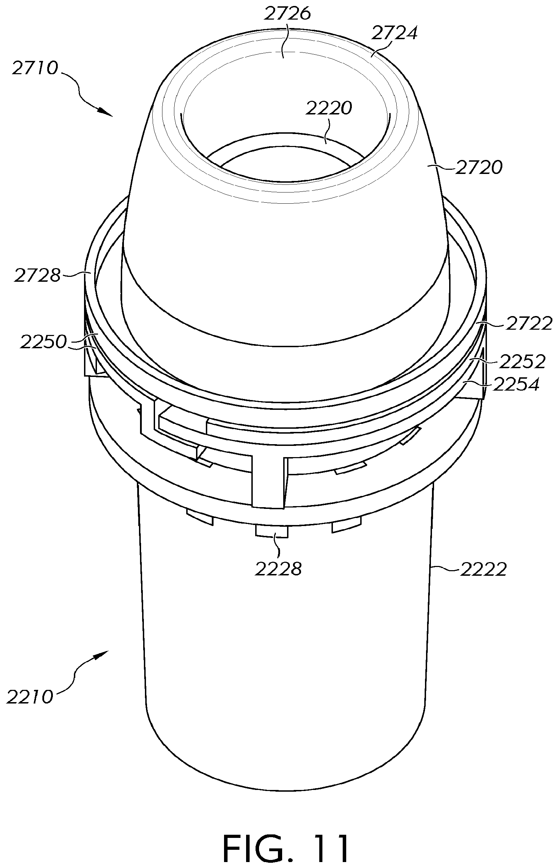

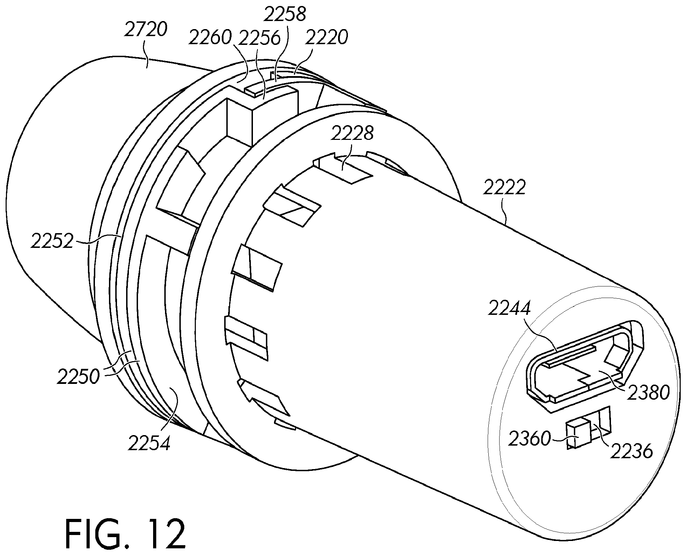

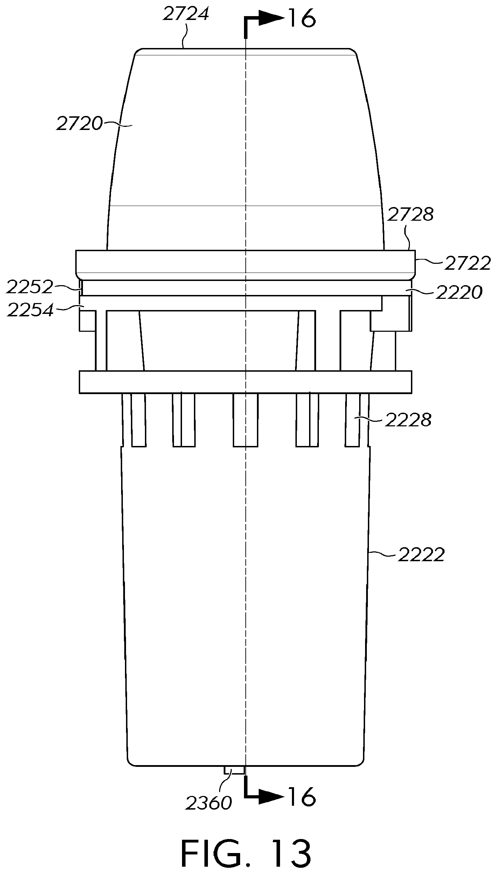

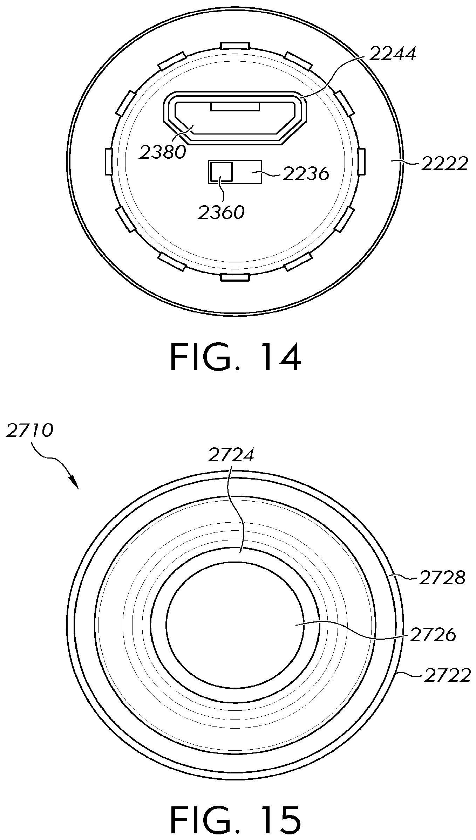

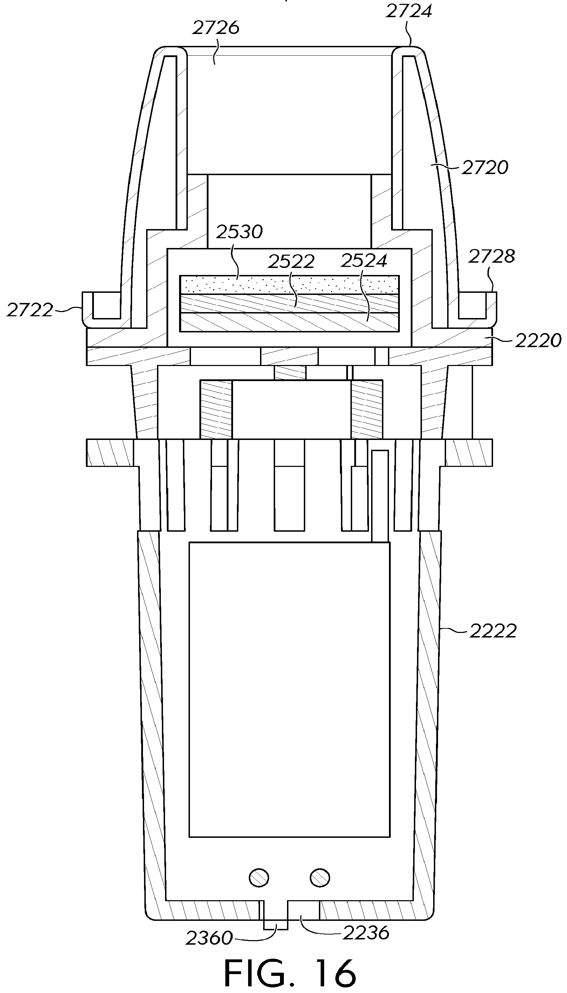

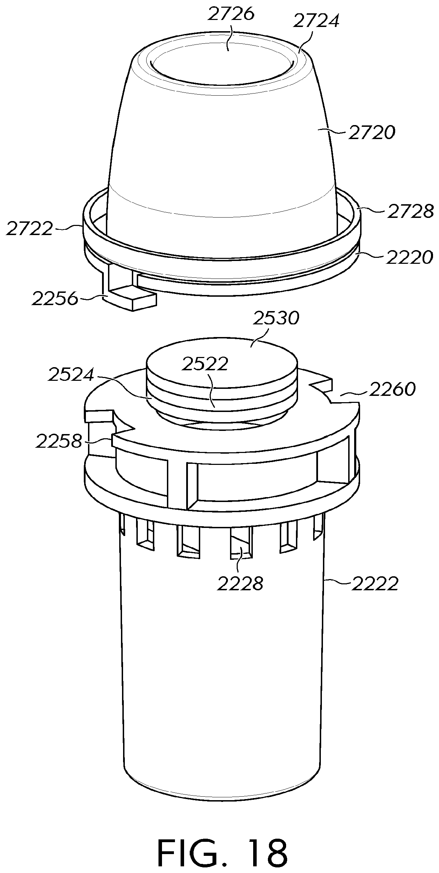

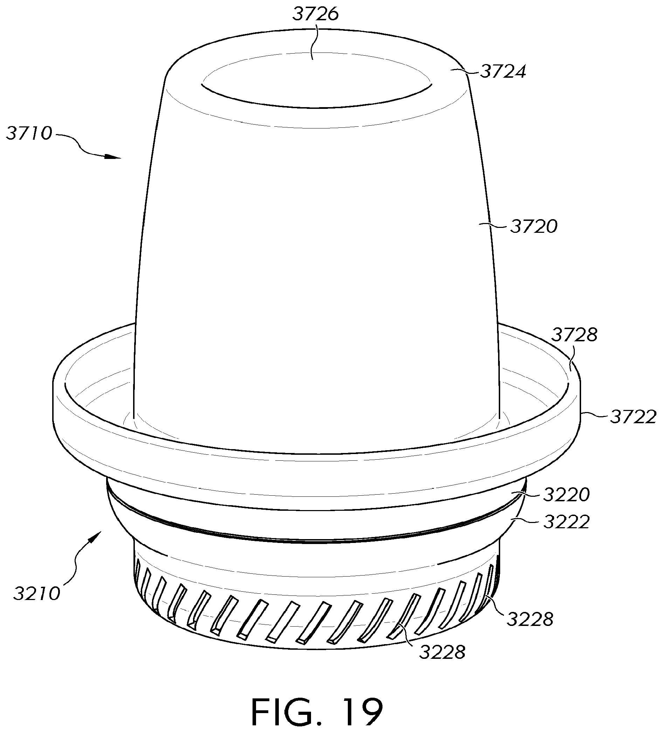

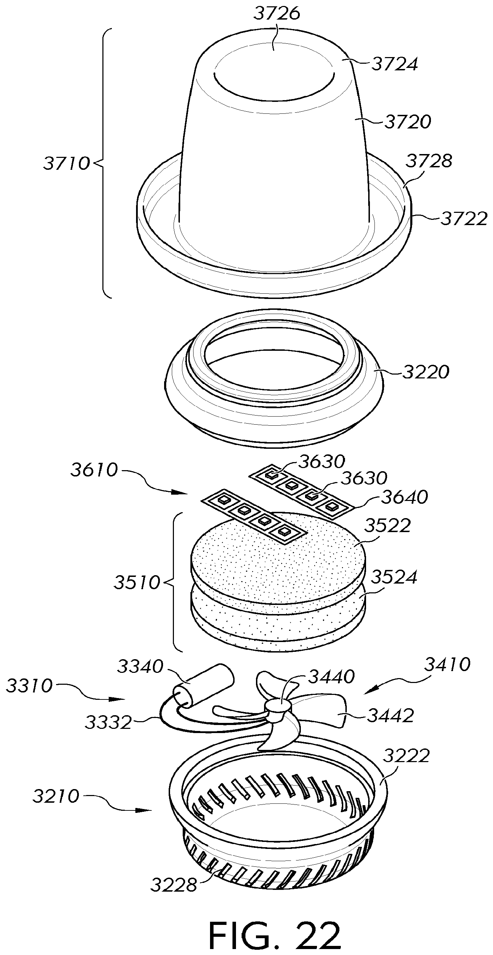

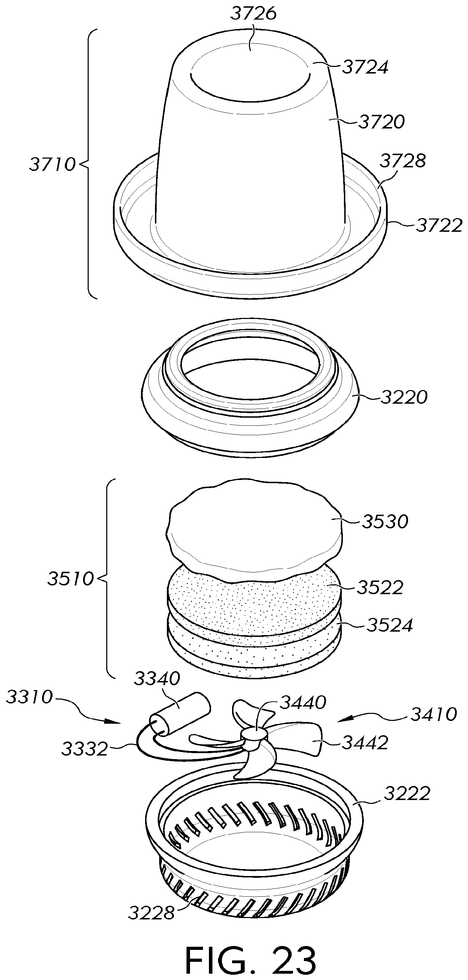

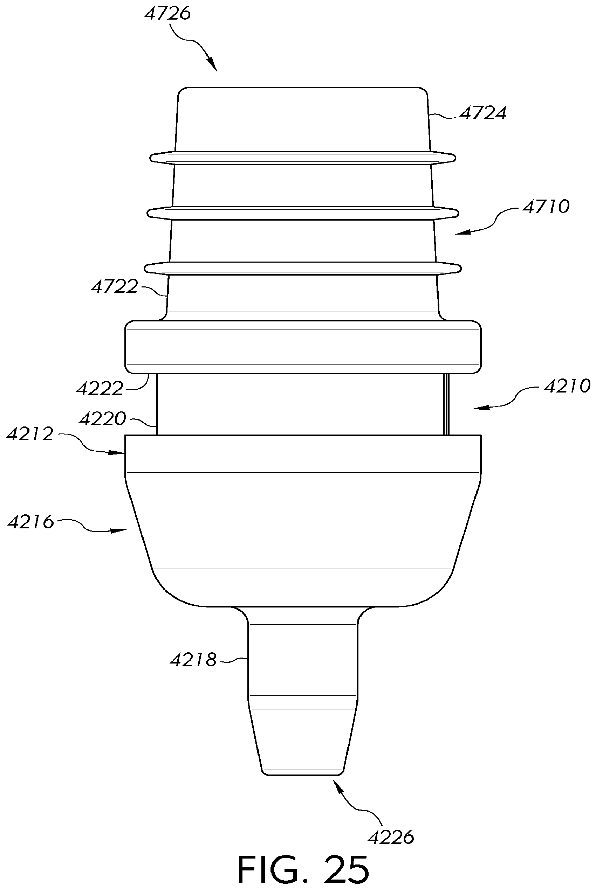

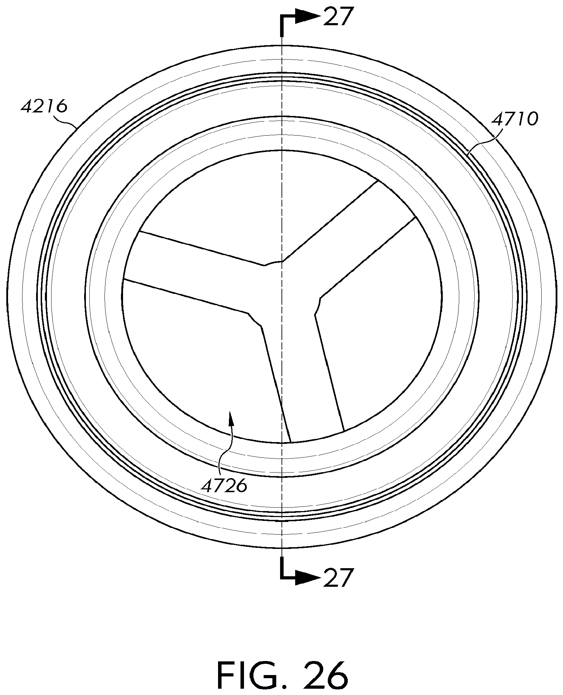

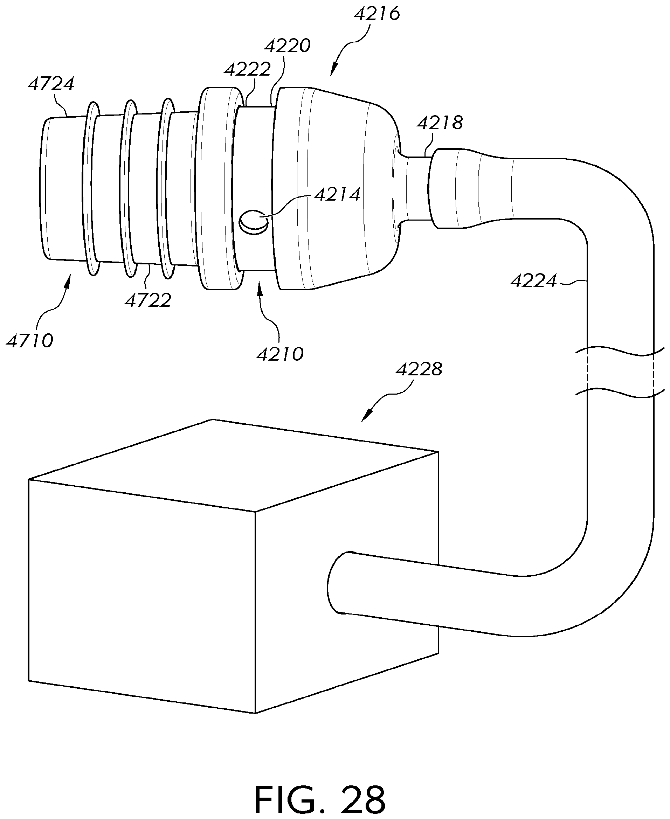

Referring now to FIGS. 1-28, an air and/or gas purification apparatus for nasal application will now be discussed in more detail. Persons skilled in the art will appreciate disclosure that enables the use of the apparatus for passage of air may additionally disclose and enable uses of the apparatus for passage of gas. The use of air in any of the following discussion, examples, and other enabling context is intended to additionally enable use for other gases in replacement of or addition to air, without limitation. The air purification apparatus for nasal application may include a housing, power component, fan component, filtration component, germicidal irradiation component, aroma component, nose plug component, monitoring component, cap, medicinal component, and additional components that will be discussed in greater detail below. The air purification apparatus for nasal application may operate one or more of these components interactively with other components for purifying and/or reducing undesirable materials from air and/or other gasses intended to enter a nasal cavity.

In the interest of clearly presenting the invention, the following disclosure will be collectively discussed in the context of various illustrative embodiments. Skilled artisans should appreciate that features discussed in the context of one embodiment may be included in additional embodiments, even if not expressly stated when discussing such embodiments, and thus should view the teachings of this disclosure as a whole and not as a collection of disparate embodiments or otherwise limiting.

The air purification apparatus will now be discussed. FIGS. 1-28 highlight examples of the air purification apparatus in various embodiments. The air purification apparatus may include various components that collectively provide an improved capability to advantageously remove pathogens, contaminants, particulates, and/or other undesirable elements from air before it may enter the respiratory system of a user. In some embodiments, the air purification apparatus may include various components located within a housing. Such components may include, without limitation, air treatment components, a power component, a nose plug component, and other components. Air treatment components may include various aspects to improve the quality of air before it is received by a user, such aspects including without limitation, a filtration component, a fan component, a germicidal irradiation component, an aroma component, a medicinal component, and/or other components that will be apparent to a person of skill in the art after having the benefit of this disclosure. Each of these components will be discussed in greater detail throughout this disclosure in the context of various embodiments. Skilled artisans should appreciate that each aspect may be included by alternative embodiments.

The housing will now be discussed in greater detail. FIGS. 1-5, 7-10, 11-14, 16-25, and 27-28 highlight examples of the housing, which may also be shown in other figures. The housing 1210, 2210, 3210, 4210 may be provided to substantially enclose an interior space within an air purification apparatus enabled by this disclosure. The housing 1210, 2210, 3210, 4210 may provide an enclosure to at least partially surround some of the functional components of an air purification apparatus within an interior space defined within the housing.

In an illustrative structural configuration, the housing 1210, 2210, 3210, 4210 may be at least partially comprised of plastic, without limitation. The housing may have virtually any diameter and height such to preserve operability of air treatment components. In one embodiment, the housing may have a substantially cylindrical shape, for example, approximately 10 millimeters and approximately 30 millimeters in diameter, without limitation. The housing may have a height sufficient to enclose the air treatment components, for example, between approximately 7 millimeters and approximately 14 millimeters, without limitation.

The cylindrical shape may advantageously correspond with a nostril of a user for improved comfort, fit, and seal upon insertion into the nostril. Additionally, the use of a cylindrical shape may advantageously assist with the removal of an air purification apparatus enabled by this disclosure when use of such apparatus is no longer desired. In additional embodiments, the housing may be provided in a non-cylindrical shape, without limitation. For example, at least part of the housing may be provided by a rectangular shape, conical shape, rounded shape, and/or another shape that would be appreciated by those of skill in the art after having the benefit of this disclosure. In the example of a rectangular shape, the housing may be between approximately 10 millimeters and approximately 30 millimeters in width and between approximately 7 millimeters and approximately 14 millimeters in height, without limitation.

The housing 1210, 2210, 3210, 4210 may be constructed using various parts, for example a top housing portion 1220, 2220, 3220, a bottom housing portion 1222, 2222, 3222, and/or other portions that will be apparent after having the benefit of this disclosure. The housing 1210, 2210, 3210, 4210 may be sized with sufficient interior space to receive, hold, and allow operation of additional components including power components 1310, 2310, 3310, air treatment components, and/or other components that may be included within the interior space.

In the same or other embodiments, the housing 1210, 2210, 3210, 4210 may additionally comprise a cap end 4220 of the housing and a nose end 4222 of the housing. The cap end 4220 may be on the opposite side of the nose end 4222 of the housing. Generally, the cap end 4220 of the housing may be near the top housing portion, while the nose end 4222 of the housing may be near the bottom housing portion. Other ends and sides of the housing 1210, 2210, 3210, 4210 may be apparent after having the benefit of this disclosure.

The housing 1210, 2210, 3210, 4210 may include access points to allow interaction with the components included within the interior space of the housing from an exterior of the housing. The access points may include a housing switch access point 1236, 2236, 3236 to allow access with a switch 1360, 2360, 3360, such as may be provided by the power component 1310, 2310, 3310. The access points may additionally include housing charging port access points 2244, 3244 to provide interaction with charging plugs and/or housing electrical contact access points 1242 to facilitate interaction with contact pins, each of which may provide electrical charge to aspects of the power component 1310, 2310, 3310 and/or additional components held within the interior space of the housing 1210, 2210, 3210, 4210.

In some embodiments, additional access points may be provided to enable additional functionality of an air purification apparatus enabled by this disclosure. Such additional access points may include sensor access points, display access points, points to access additional interactive elements related to the air treatment components and/or the power components, and otherwise.

In various embodiments, the housing may further comprise an auxiliary aperture 4214 on the housing 1210, 2210, 3210, 4210. The auxiliary aperture 4214 may be located on the wall of the housing. Additionally, the auxiliary aperture 4214 may be of any size. Those skilled in the art may realize the size and location of the auxiliary aperture after having the benefit of this disclosure. Furthermore, the auxiliary aperture 4214 may hold various purposes for the apparatus, including, but not limited to, pressure relief, moisture relief, an access point, and/or other purposes that a person skilled in the art would realize after having the benefit of this disclosure. Some of these purposes will be discussed in greater detail throughout this disclosure.

As previously mentioned, the auxiliary aperture 4214 may be used for moisture relief. In various embodiments, the housing 1210, 2210, 3210, 4210 may additionally include a drying agent. The drying agent may at least partially remove moisture within the interior space of the housing 1210, 2210, 3210, 4210. In the same or different embodiments, the moisture collected by the drying agent may be at least partially vented out through the auxiliary aperture 4214. Drying agents include, but are not limited to, calcium chloride, calcium oxide, sodium sulfate, sulphuric acid, magnesium sulfate, and/or other drying agents. Those skilled in the art will appreciate the various types of drying agents, whether chemical or mechanical, that may be used in the apparatus, after having the benefit of this disclosure.

The housing 1210, 2210, 3210, 4210 may additionally include housing inlets 1228, 2228, 3228 through which air may pass from an exterior location respective to the housing to the interior space substantially enclosed by the housing. For example, the housing 1210, 2210, 3210, 4210 may include numerous housing inlets 1228, 2228, 3228 at a bottom location with respect to the housing, such as near the lower end of the bottom housing portion 1222, 2222, 3222. This configuration may be advantageous wherein the internal space is organized such that air may pass through aspects of the power components 1310, 2310, 3310 without substantial restriction and be received by additional air treatment components before being presented to the nasal cavity of a user.

In another embodiment, the housing inlets 1228, 2228, 3228 may be provided near an upper location of the bottom housing portion 1222, 2222, 3222. This configuration may be advantageous wherein the interior space is organized such that the lower part of the bottom housing portion 1222, 2222, 3222 is substantially filled with power components 1310, 2310, 3310 such that airflow is maximized by entering the interior space of the housing 1210, 2210, 3210, 4210 above the location at which the power components are stored.

The housing inlets 1228, 2228, 3228 may be configured at various angles, which may affect the flow characteristics of the air entering the interior space of the housing 1210, 2210, 3210, 4210 from an exterior location. For example, the housing inlets 1228, 2228, 3228 may be configured with an angled orientation, which may improve the flow characteristics of air circulating within the interior space of the housing 1210, 2210, 3210, 4210, such as may be moved by a fan component 1410, 3410. In other embodiments, the housing inlets 1228, 2228, 3228 may be provided in substantially vertical orientation, which may advantageously provide flow characteristics to improve circulation for alternative fan configurations that would be apparent to those of skill in the art after having the benefit of this disclosure. Skilled artisans will appreciate additional configurations of housing inlets 1228, 2228, 3228 including horizontal, curved, angled, slotted, mixed, and otherwise configured orientations, without limitation.

In some embodiments, the bottom housing portion 1222 may be provided in multiple pieces. For example, the bottom housing portion 1222 may be provided having a bottom housing portion first piece 1224 and a bottom housing portion second piece 1226. The bottom housing portion first piece 1224 may be received by and substantially held by the bottom housing portion second piece 1226. For example, the bottom housing portion first piece 1224 may include one or more housing male connection members 1232 and the bottom housing second piece 1226 may include one or more housing female connection members 1234. The housing male connection members 1232 on the bottom housing portion first piece 1224 may be oriented such to correspond with the housing female connection members 1234 on the bottom housing portion second piece 1226. In this configuration, the housing male connection members 1232 may be inserted into the housing female connection members 1234 such to substantially hold both the bottom housing portion first piece 1224 and the bottom housing portion second piece 1226 together while coupled.

In some embodiments, the housing 1210 may include guideways to facilitate insertion of the housing 1210 into a case 1810. The case 1810 will be discussed in greater detail later in this disclosure. The guideways may advantageously assist the housing 1210 to be received by the case 1810 in a preferred orientation. For example, a case 1810 may be provided that includes electrical contacts 1844 capable of providing electrical power to an air purification apparatus enabled by this disclosure with corresponding electrical contacts 1370. In this example, the guideways may assist with orienting the housing 1210 such as to align the case 1810 and apparatus charging contacts to permit the transfer of electrical power. In additional embodiments, the guideways may include features to assist with improving a secure reception of the air purification apparatus enabled by this disclosure within the case 1810 upon insertion.

In another embodiment, the bottom housing portion 1222, 2222, 3222 may include a housing switch access point 1236, 2236, 3236 and/or a housing charging port access point 2244, 3244 upon its bottom surface. In this embodiment, a switch 1360, 2360, 3360 and/or charging port 2380, 3380 provided by the power component 1310, 2310, 3310 may be at least partially passed through the access points such that they may be interacted with by a user. For example, wherein a housing switch access point 1236, 2236, 3236 is included, a switch 1360, 2360, 3360 provided by the power component 1310, 2310, 3310 may be passed through the housing switch access point 1236, 2236, 3236 such that sufficient material of the switch 1360, 2360, 3360 is accessible for manipulation by a user. In another example, wherein a housing charging port access point 2244, 3244 is provided, a charging port 2380, 3380 may be at least partially passed through the house charging port 2380, 3380 access point such that it may receive a charging cable and/or other cable that may permit both the transfer of electrical power and/or data.

The housing 1210, 2210, 3210, 4210 may additionally include an interface to receive and/or hold a nose plug component 1710, 2710, 3710, 4710, which may be distal to the bottom surface of the housing. The nose plug component 1710, 2710, 3710, 4710 will be discussed in greater detail later in this disclosure. In this embodiment, the housing 1210, 2210, 3210, 4210 may include features to apply friction to the nose plug component 1710, 2710, 3710, 4710, textured aspects or protrusions to receive corresponding depressions on an interior surface of the nose plug component 1710, 2710, 3710, 4710, or otherwise be configured to operatively hold the nose plug component 1710, 2710, 3710, 4710 to the housing 1210, 2210, 3210, 4210 in a removably installed configuration. Additionally, in the same or other embodiments, the nose plug component 1710, 2710, 3710, 4710 may include textured aspects, protrusions, or ridges on the exterior which may assist in applying friction to at least part of the nostril when the apparatus is in use. In an exemplary embodiment, the nose plug component 1210, 2210, 3210, 4210 may include three circumferential ridges, without limitation. However, those skilled in the art may appreciate the various number of ridges that may be included on the nose plug component, after having the benefit of this disclosure.

In some embodiments, the housing 2210 may include a locking interface 2250. In some examples of an embodiment with a locking interface, the locking interface 2250 may serve as at least part of the housing 2210, such as being included by the top housing portion 2220. In other examples of an embodiment with a locking interface, the locking interface 2250 may be distinct and separate from the top housing portion 2220. Those of skill in the art will appreciate the inclusion of these various examples as nonlimiting.

The locking interface 2250 may include a locking interface top portion 2252 and a locking interface bottom portion 2254. The locking interface top portion 2252 may include a locking member 2256 that protrudes downward such as to be received by the locking interface bottom portion 2254. The locking interface bottom portion 2254 may include a locking lip 2258 that runs at least partially around the circumference of the locking interface bottom portion 2254. The locking lip 2258 may include a locking gap 2260 with sufficient width such that the locking member 2256 of the locking interface top portion 2252 may be passed through the locking gap 2260.

The distance between the lower portion of the locking member 2256 may be configured such that, once passed through the locking gap 2260, it may be rotated in a first locking direction to receive the locking lip 2258 and configure the locking interface 2250 in a locked position. Once configured in the locked position, the locking member 2256 may be substantially restricted in latitudinal movement by its reception of the locking lip 2258 and substantially limit access to the interior space provided by the housing 2210.

Additionally, the locked position may be reversible by rotating the locking interface top portion 2252 in a second locking direction such as to release the locking lip 2258 from the locking member 2256 and configure the locking interface 2250 and an unlocked position. Once configured in the unlocked position, the locking member 2256 may be withdrawn through the locking gap 2260 to provide access to the interior space otherwise enclosed by the housing 2210.

In some embodiments, the locking interface top portion 2252 may include a ring of material that extends at least partially around the circumference of the locking interface top portion 2252. If included, the ring of material provided by the locking interface top portion 2252 may advantageously assist with inserting the locking member 2256 within the locking gap 2260 to a sufficient depth to correspond with the locking lip 2258.

In the same or other embodiments, the air purification apparatus may additionally include a cap 4216. Those skilled in the art may realize that the apparatus may be used with or without the cap. However, the cap 4216 may advantageously provide a male fitting for a delivery line to a gas source. The delivery line and gas source will be discussed in greater detail below. The cap 4216 may be removably attached to the housing 4210 via a cap receiving feature 4212 of the housing 4210, which will be discussed in greater detail below. Generally, the cap 4216 may assist in direct administration of gas, via a gas receiving aperture 4226, into at least part of the housing 4210. In an illustrative structural configuration, the aperture may be located virtually anywhere on the cap. In some embodiments, the aperture may be located on the opposite side of the cap receiving feature 4212. In the same or other embodiments, the gas receiving aperture 4226 may be located on the cannula 4218, which will be discussed in greater detail below. Additionally, the gas receiving aperture may be of virtually any shape and of any size, as will be appreciated by those of skill in the art, after having the benefit of this disclosure.

In one or more embodiments, the cap 4216 may additionally comprise a cannula 4218. In some embodiments, the cannula may be located on one end of the cap 4216. The cannula 4218 may have a cannula diameter. The cannula 4218 may have a cannula male end which may advantageously be used to fit into the end of a delivery line 4224. The delivery line 4224 may have a delivery line diameter. The delivery line may be used to connect the cannula 4218 to a gas source 4228. In embodiments, the cannula diameter may be smaller than the delivery line diameter.

In an illustrative structural configuration, the cap may be at least partially comprised of plastic or rubber, without limitation. The cap may have virtually any diameter, circumference, and height such to preserve operability of any components inside. In one embodiment, the cap may have a diameter of approximately 0.576 inches, without limitation. In another or same embodiment, the height of the cap and cannula combination may be approximately 0.608 inches, without limitation. In the same or different embodiments, the height of the cannula may be approximately 0.300 inches, without limitation.

In embodiments, the apparatus may additionally include a gas source 4228 from which gas may be released from or entered into. Examples of a gas source 4228 may include a pressurized tank or a continuous positive airway pressure machine, without limitation. Additionally, the gas source 4228 may be attached to a delivery line 4224. The delivery line 4224 may advantageously assist in delivering gas from the gas source 4228 to the apparatus. As previously mentioned, the delivery line 4224 may have a delivery line diameter. The delivery line may be selectively installed to the cap, so that the gas from the delivery line may be received through the gas receiving aperture 4226 of the cap 4216. In various embodiments, the delivery line 4224 may be made of plastic or rubber, without limitation. Furthermore, the air inside the delivery line 4224 may enter the cannula 4218 via the gas receiving aperture 4226. As the gas continues from the cannula 4218 into the cap 4216, the effective diameter of the cap may increase. This increase of the diameter may also increase the efficacy of filtration when the air is delivered through the filtration component 4510. Those people skilled in the art will appreciate the various benefits that one may find from an increase in the diameter from the delivery line to the cannula and to the cap.

In embodiments, the housing 4210 may comprise a cap receiving feature 4212 which may advantageously assist in temporarily locking the cap 4216 to the housing 4210. In the various embodiments, the cap receiving feature 4212 may be at least partially flexible to allow for installation and/or removal of the cap 4216. The cap receiving feature 4212 may be provided near the cap end 4220 of the housing 4210. In various embodiments, the cap receiving feature 4212 includes a circumferential ridge around the cap end 4220 of the housing 4210. In other embodiments, the cap receiving feature 4212 includes a partial rim on one or more sides of the cap end 4220 of the housing 4210. Those of skill in the art will appreciate the various mechanical structures that may be included in the cap receiving feature 4212 to allow for the temporary installation and/or removal of the cap 4216 on the housing 4210 or any other part of the apparatus.

The power component will now be discussed in greater detail. FIGS. 1-5, 7-8, 12-14, 16-17, and 22-23 highlight examples of the power component, which may also be shown in other figures. The power component 1310, 2310, 3310 may be located substantially within the interior space to provide electrical power to the air treatment components. The power components 1310, 2310, 3310 may advantageously assist with the conditioning, storage, and provision of electrical power to the various components of an air purification apparatus enabled by this disclosure. For example, the power components 1310, 2310, 3310 may provide electrical power to the various air treatment components held within the interior space provided by the housing 1210, 2210, 3210, 4210. The power component 1310, 2310, 3310 may include power delivery electronics 1330, a battery 1340, 2340, 3340, battery charging electronics 1350, and other aspects to assist with the transmission and delivery of electrical power.

The power component 1310, 2310, 3310 may include a battery 1340, 2340, 3340. In some embodiments, the battery may be rechargeable. In other embodiments, the battery may be removable from the interior space and replaceable. The battery 1340, 2340, 3340 may be virtually any size that may fit within the housing 1210, 2210, 3210, 4210. For example, the battery may be substantially cylindrical without limitation. In one example configuration, the battery may be between approximately 14 millimeters and approximately 20 millimeters in length and between approximately 5 millimeters and approximately 8 millimeters in diameter, without limitation.

Those of skill in the art will appreciate various additional configurations of batteries that may be used with an air purification apparatus, such as one enabled by this disclosure. Illustrative batteries may include lithium-ion batteries, nickel metal hydride batteries, rechargeable batteries, replaceable batteries, and/or other battery types that would be appreciated by those of skill in the art, without limitation.

The power component 1310, 2310, 3310 may include battery charging electronics 1350. In one example, the battery charging electronics 1350 may receive external power from an external power source, condition the external power to a charging power receivable by the battery 1340, 2340, 3340, and store at least part of the charging power in the battery for use by the air treatment components as the electrical power. The power component 1310, 2310, 3310 may control the application of the charging power to the battery 1340, 2340, 3340 to improve the charge state of the battery while advantageously decreasing the likelihood that the battery should become overcharged. Skilled artisans will appreciate various configurations of battery charging electronics 1350 which are intended to be within the scope and spirit of this disclosure. Skilled artisans will appreciate additional battery charging electronics and other features may be included by the power component 1310, 2310, 3310 to monitor the charge state of the battery, number of charge cycles applied to the battery, number of discharge cycles applied to the battery, battery temperature, battery voltage, irregularities detected within or with regard to the battery, and other features that may be provided by the battery charging electronics, without limitation.

The power component 1310, 2310, 3310 may additionally include power delivery electronics 1330 to provide electrical power from the battery 1340, 2340, 3340 to various components included within the interior space of an air purification apparatus enabled by this disclosure. The power delivery electronics 1330 may advantageously control the flow of electrical power to the various treatment components that operate by using the electrical power. For example, the power delivery electronics 1330 may control the power state of a fan component 1410, 3410, the operational speed of the fan included by the fan component, operation of a UV light source 3630 included by a germicidal irradiation component 3610, the duty cycle of such UV light source 3630, such as if provided as an LED, and other power-related operational variables that would be appreciated by those of skill in the art after having the benefit of this disclosure.

The power component 1310, 2310, 3310 may include electrical wires 1332, 3332, which may facilitate the transmission of electrical power from the various aspects included by the power component. Skilled artisans will appreciate additional configurations in which the electrical wires 1332, 3332 may be at least partially replaced by electrical contacts 1370, electrical traces provided in a circuit board, and/or otherwise, without limitation.

The power component 1310, 2310, 3310 may include a switch 1360, 2360, 3360 that may be toggled by a user to enable or disable the application of electrical power to the various air treatment components included within the interior space of an air purification apparatus enabled by this disclosure. The switch 1360, 2360, 3360 may be toggled between various states, which may include without limitation and on state and an off state. In some embodiments, the switch 1360, 2360, 3360 may include additional states such as to control selective engagement of various air treatment components that rely on electrical power delivered by the power component 1310, 2310, 3310. In additional embodiments, the switch 1360, 2360, 3360 may be operable through sensors that detect touch. In other embodiments, the switch may be provided as a continuous selection of electrical power application, as will be appreciated by those of skill in the art. In various embodiments, the switch 1360, 2360, 3360 and other power control mechanisms may be located virtually anywhere with respect to the housing 1210, 2210, 3210, 4210 if such placement does not interfere with the operability of air treatment components.

The power component 1310, 2310, 3310 may include interfaces to allow the battery charging electronics 1350 to receive the external power from the external power source. In one example, such interfaces may include electrical contacts 1370. In this example, the electrical contacts 1370 may be configured to orient with corresponding electrical contacts from the external power source, for example, a case 1810 having case electrical contacts 1844. In this example, once aligned, the electrical contacts 1370, 1844 may permit the flow of the external electrical power from the external power source through the electrical contacts 1370 and to the battery charging electronics 1350 before being stored in the battery 1340.

In another example, the interface allowing the battery charging electronics to receive the external power may include a charging port 2380, 3380. In some embodiments, the charging port 2380, 3380 may be provided as a USB-based port. Examples of USB-based ports may include USB Type-A, USB Type-B, micro USB, USB Type-C, and other USB standards that would be apparent to those of skill in the art. Additionally, those skilled in the art will appreciate additional plugs and corresponding ports capable of delivering electrical power that are intended to be included within the scope of the disclosure, without limitation.

In some embodiments, the charging port 2380, 3380 and corresponding charging cable may be configured to enable data transmission in addition to transmission of external power. In this embodiment, the functionality of an air purification apparatus enabled by this disclosure may be controlled, modified, updated, or otherwise affected depending on the data transmitted through the charging port 2380, 3380. In another example, a monitoring component provided by an air purification apparatus enabled by this disclosure may transmit data from such an air purification apparatus to an external computing device such as to perform analytics on conditions sense by sensors, operational logs, battery health, and/or other information that may be detected, stored, and made available by a monitoring component and/or other aspects of an air purification apparatus enabled by this disclosure. The data transmission may establish wired connectivity with a separate electronic device, such as a console capable of analyzing information transmitted to it regarding presence or absence of virus in a user utilizing an air purification apparatus such as, without limitation, presence, or absence of COVID-19 virus.

The air treatment components will now be discussed generally. Various components included by the air treatment components will be discussed in greater detail throughout this disclosure. Air treatment components may interact with air flowing through an interior space of an air purification apparatus, such as one enabled by this disclosure, to change a characteristic of such air. For example, the air treatment components may include a filtration component 1510, 2510, 3510, 4510 to remove particulates, pathogens, microorganisms, and other undesirable elements from the air as it passes through the interior space. In another example, a germicidal irradiation component 3610 may at least partially sterilize aspects of the air passing through the interior space. These and other components included by the air treatment components will be discussed in greater detail below.

The fan component will now be discussed in greater detail. FIGS. 1, 7-8, and 20-23 highlight examples of the fan component, which may also be shown in other figures. The fan component 1410, 3410 may be provided to assist with the movement of air throughout the interior space of an air purification apparatus, such as one enabled by this disclosure, during operation. The fan component 1410, 3410 may include a motor 1420, a fan 1440, 3440 having fan blades 1442, 3442, and optionally a fan shroud 1444. The motor 1420 may be operatively attached to the power delivery electronics 1330 and/or the battery 1340, 2340, 3340 to receive electrical power that may be used to drive the motor 1420. The speed at which the motor 1420 is operated may be at least partially controlled by the power delivery electronics 1330, for example by controlling the voltage applied to the motor, by controlling the frequency of pulses delivered to a pulse width modulation motor, or otherwise controlled as will be appreciated by those of skill in the art.

The fan 1440, 3440 may be oriented such as to draw air from the housing inlets 1228, 2228, 3228. This drawn air may be at least partially moved by the rotating fan blades 1442, 3442 provided by the fan 1440, 3440 and moved through, over, past, or otherwise in relation to other air treatment components included within the interior space. The fan component 1410, 3410 may advantageously provide active pressure within the interior space to direct the air through filters provided by a filtration component 1510, 2510, 3510, 4510, past UV light sources 3630 provided by a germicidal irradiation component 3610, aromatic material provided by an aroma component 2530, 3530, and/or otherwise as may be provided by the air treatment components included within the interior space of an air purification apparatus enabled by this disclosure.

In one embodiment, the fan blades 1442 may be at least partially enclosed by a fan shroud 1444. In this embodiment, the fan shroud 1444 may assist with controlling the direction and movement of air passing by the fan blades. In some embodiments, the fan shroud 1444 may be included by at least part of the housing 1210. For example, the fan shroud 1444 may be built into at least part of the top housing portion 1220. In other embodiments, the fan shroud 1444 may be provided as a distinct member included within the interior space defined within the top housing portion 1220, 2220, 3220 and the bottom housing portion 1222, 2222, 3222. In some embodiments, the fan shroud 1444 may include an interior diameter such as corresponding with filters provided by the filtration component 1510, 2510, 3510, 4510. Those of skill in the art will appreciate additional advantageous applications of a fan shroud 1444 should it be included in such embodiments.

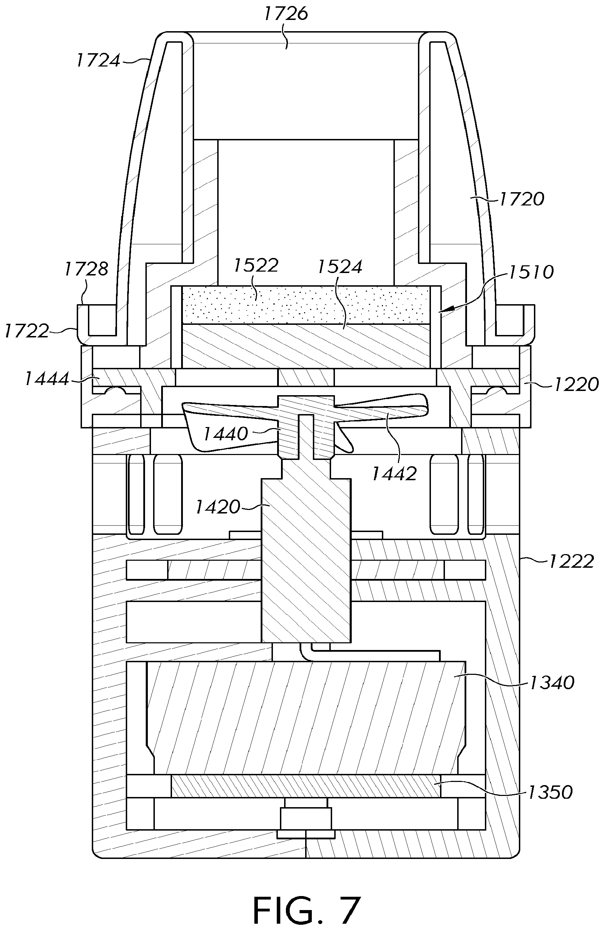

The filtration component will now be discussed in greater detail. FIGS. 7-8, 16-18, 20-23, and 27 highlight examples of the filtration component, which may also be shown in other figures. The filtration component 1510, 2510, 3510, 4510 may include various filters and or other elements to affect the composition of air passing through or around the filtration component. The filtration component may also have a filtration diameter. In some embodiments, the filtration diameter may be larger than the delivery line diameter of the delivery line 4224. As previously mentioned, the increase of diameter inside the cap 4216 may advantageously increase a filtration efficacy of the filtration component 4510, without limitation. Generally, air passed through the filtration component 1510, 2510, 3510, 4510 may be directed to the nose plug component. In one embodiment, the filtration component 1510, 2510, 3510, 4510 may include a carbon filter 1524, 2524, 3524 to advantageously remove undesired particulates, impurities, including microorganisms, and other materials from air passing through such carbon filter. In one example, provided without limitation, a carbon filter may be provided with a thickness of between approximately 5 millimeters and approximately 10 millimeters. The air may be passed through the carbon filter 1524, 2524, 3524 by active pressure applied by the fan component 1410, 3410 discussed above. In some embodiments, the carbon filter may include activated carbon, as will be appreciated by those of skill in the art.

The carbon filter 1524, 2524, 3524 may reduce the presence of airborne pathogens, including viruses and bacteria, from entering the respiratory system. The carbon filter 1524, 2524, 3524 may filter gaseous volatile organic compounds and other undesirable substances, such as cigarette smoke, drying paint, benzene, toluene, xylene, and/or other substances, without limitation. Carbon filtration may additionally filter unpleasant odors from air and may contribute to decreased stress and increased calmness and feelings of well-being on the part of such user. The carbon filter 1524, 2524, 3524 may include activated carbon.

In another embodiment, the filtration device may include a high efficiency particulate absorbing (HEPA) filter, as will be appreciated by those of skill in the art. In one example, provided without limitation, a HEPA filter may be provided having a wafer configuration with a thickness of between approximately 5 millimeters and approximately 10 millimeters. The HEPA filter 1522, 2522, 3522 may reduce potentially harmful agents and contaminants such as air bacteria, dust, pollen, mold, and other types of airborne particles. As will be appreciated by those of skill in the art, HEPA filtration may remove at least approximately 98% of airborne contaminants having a size of approximately 0.3 microns or more. Additionally, the HEPA filter may be replaceable by the user.

In some embodiments, either the carbon filter 1524, 2524, 3524 or the HEPA filter 1522, 2522, 3522 may be provided as the sole filtering technique included by the filtering component. In other embodiments, a two-stage filtering process may be provided whereby air is moved using the fan through the carbon filter 1524, 2524, 3524 for a first filtering pass and then through the HEPA filter 1522, 2522, 3522 for an additional filtering pass. In some embodiments, the filter may be scented or otherwise include and/or provide a substance capable of interacting with an olfactory nerve, without limitation. Those of skill in the art will appreciate additional filter types that may be included, which may also be arranged by the filtering component to affect the removal of particulates, pathogens, and other undesirable elements from the air passing through the filtration component 1510, 2510, 3510, 4510.

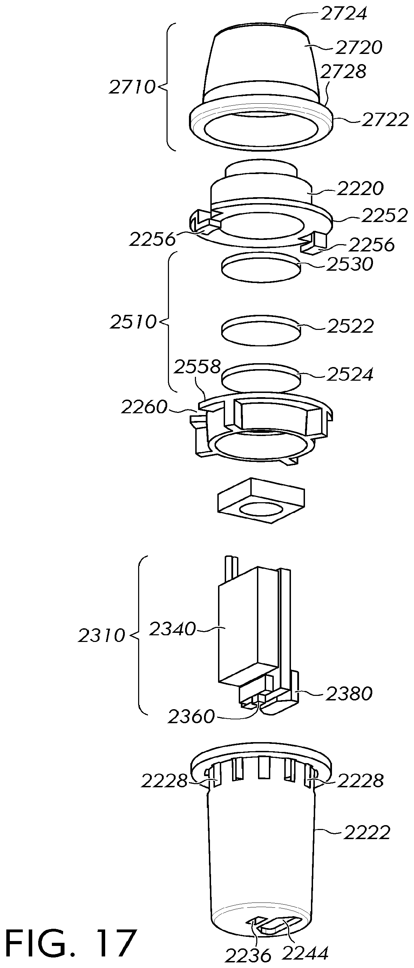

The aroma component will now be discussed in greater detail. FIGS. 17-19 and 24 highlight examples of the aroma component, which may also be shown in other figures. Inclusion of an aroma component 2530, 3530 may advantageously enhance a user's feeling of well-being and calmness, thus decreasing a user's stress. The aroma component may, without limitation, be located within a housing 2210, 3210 as contemplated herein. The aroma component may be located subsequent to the filtration component 2510, 3510, such as to receive the air after it has passed through the filtration component.

In one embodiment, an aroma component 2530, 3530 may be included such as to improve the aroma of the air passing through the interior space. The aroma component 2530, 3530 may advantageously apply a pleasurable or otherwise desirable scent to the air, increasing the mood and psychological well-being of a user operating an air purification apparatus such as one enabled by this disclosure. In another embodiment, the aroma component 2530, 3530 may provide essential oils, congestion relief solutions, and/or other substances that may be added to the air prior to being sent to the nasal cavity of the user.

The aroma component 2530, 3530 may include an aroma reservoir to hold an aromatic material capable of providing an aroma. An illustrative aroma reservoir may include porous materials, sponges, sponge-like materials, wafers, meshes, absorbent materials, liquid basins, and other materials or containers that would be apparent to a person of skill in the art. Without limitation, an illustrative aroma reservoir constructed as a porous material may have a thickness of between approximately 5 millimeters and approximately 10 millimeters. The aromatic material may be a perfume, essential oil, aromatic solution, natural ingredient, synthetic ingredient, and/or another material that would be appreciated by those of skill in the art after having the benefit of this disclosure.

The aroma component 2530, 3530 may be configured such that upon application of positive pressure by the fan component 1410, 3410, air passes through the interior space of an air purification apparatus such as one enabled by this disclosure sufficiently to come in contact with the aroma reservoir, thus transferring at least part of the aroma provided by the aromatic material to the air. The aromatic air may then pass through the nose component and into the nasal cavity of a user engaging the olfactory receptors and providing a psychological and/or satisfactory response to the user.

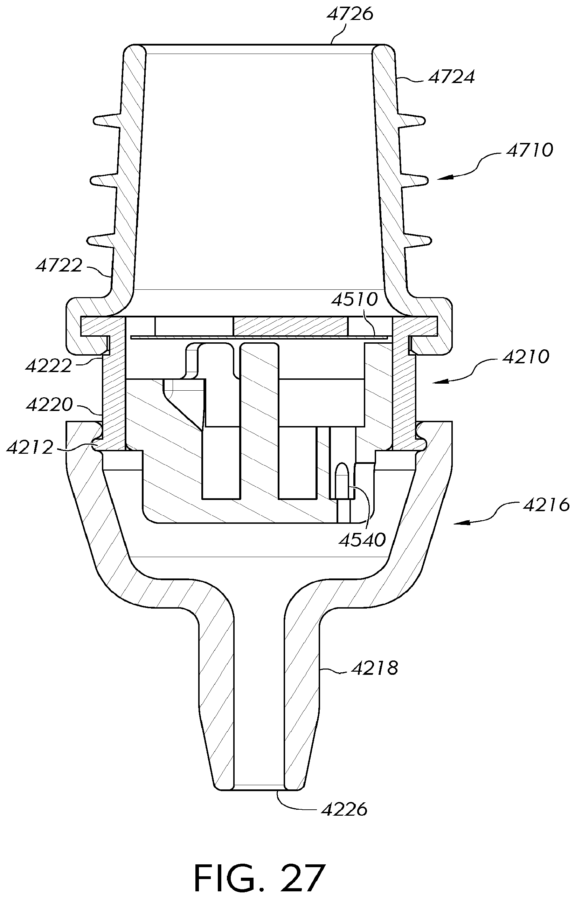

The medicinal component will now be discussed in greater detail. FIG. 27 highlights an example of the medicinal component, which may also be shown in other figures. Inclusion of a medicinal component 4540 may advantageously enhance a user's health, thus decreasing a user's susceptibility to reacting negatively to various symptoms. The medicinal component may, without limitation, be located within a housing 4210 as contemplated herein. In most embodiments, the medicinal component may be located subsequent to the filtration component 4510, such as to receive the air after it has passed through the filtration component. In other embodiments, the medicinal component may be located before the filtration component 4510, in the housing 4210.

In one embodiment, a medicinal component 4540 may be included such as to introduce medicine into gas passing through the interior space. The medicinal component 4540 may advantageously apply various medicines in different amounts and/or in different forms, such as an aerosol, without limitation. Such medicines may include drugs suitable for intranasal administration, which may include, without limitation, antiepileptics, sedatives, opiate analgesics and antagonists, local anesthetics, epistaxis control agents, and anti-hypoglycemic agents.

The medicinal component 4540 may include a medicinal reservoir to hold a medicine. An illustrative medicinal reservoir may include porous materials, sponges, sponge-like materials, wafers, meshes, absorbent materials, liquid basins, and other materials or containers that would be apparent to a person of skill in the art. Without limitation, an illustrative medicinal reservoir constructed as a porous material may have a thickness of between approximately 5 millimeters and approximately 10 millimeters. The medicinal material may be a natural ingredient, synthetic ingredient, a medicinal solution, and/or another material that would be appreciated by those of skill in the art after having the benefit of this disclosure.

In one embodiment, the medicinal component 4540 may be configured such that upon application of positive pressure by the gas source 4228, gas passes through the interior space of an air purification apparatus such as one enabled by this disclosure sufficiently to come in contact with the medicinal reservoir, thus transferring at least part of the medicine provided by the medicinal material to the gas. The medicinal air may then pass through the nose plug component and into the nasal cavity of a user.

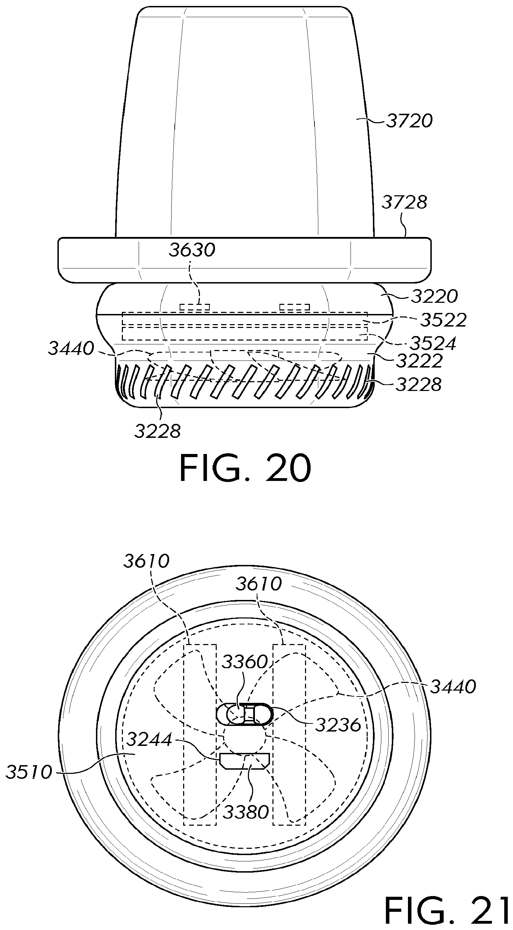

The germicidal irradiation component will now be discussed in greater detail. FIGS. 21-23 highlight examples of the germicidal irradiation component, which may also be shown in other figures. The germicidal irradiation component 3610 may emit ultraviolet electromagnetic radiation that at least partially sterilizes pathogens from the air prior to entry into the nasal cavity, such as hazardous microorganisms, bacteria, and viruses.

The germicidal irradiation component 3610 may be configured to emit electromagnetic energy capable of at least partially sterilizing materials included in the air passing through and contacting the electromagnetic energy. In the interest of simplicity in this disclosure, the electromagnetic energy will be discussed in the context of ultraviolet light, without limitation. Those of skill in the art will appreciate additional electromagnetic energy that may additionally provide disinfecting and/or germicidal benefits that are intended to be included within the scope of this disclosure.

The germicidal irradiation component 3610 may include a light source capable of emitting light having frequencies that facilitate the sterilization of undesired contaminants, pathogens, and other elements in the air passing by the germicidal irradiation component 3610. The light source may be an ultraviolet light source that may emit light in the frequency range within the ultraviolet spectrum, as will be appreciated by those having skill in the art. In some embodiments, the ultraviolet light source may include light emitting diodes (LEDs) 3630 that emit or otherwise provide light in the ultraviolet spectrum. In some embodiments, multiple ultraviolet light sources may be included on a substrate 3640, for example, a circuit board onto which the ultraviolet light sources 3630 are installed.

The ultraviolet light sources 3630, for example via the substrate 3640, may be powered at least partially by the power delivery electronics 1330 of the power component 3310. In some embodiments, the ultraviolet light source may be provided as a dimmable and/or otherwise controllable configuration. For example, the brightness and power applied to the ultraviolet light source may be at least partially controlled by affecting the duty cycle at which the light source operates. The duty cycle may be controlled by the power delivery electronics, other aspects included by the germicidal irradiation component 3610, and/or the power component 3310, without limitation.

In some embodiments, the germicidal irradiation component 3610 may include multiple substrates 3640, each of which including multiple ultraviolet light sources 3630 capable of emitting a sufficient amount of ultraviolet light such as to substantially sterilize contaminants included in the air passing by the germicidal irradiation component 3610. The intensity of light emitted by the ultraviolet light sources 3630 may be controlled with respect to the number of ultraviolet light sources included by the germicidal irradiation component 3610. For example, a number of high intensity ultraviolet light sources may be provided by the germicidal irradiation component 3610 to allow a high degree of sterilizing light to be emitted by selected number of ultraviolet light sources. In another example, a larger quantity of ultraviolet light sources may provide sterilizing light at a lower intensity, but collectively providing a sufficient amount of sterilizing light over a wider area of application from the larger number of ultraviolet light sources.

In some embodiments, the germicidal irradiation component 3610 may include additional light emitting sources and/or other electromagnetic energy emitting sources to further sterilize aspects of the air passing by the germicidal irradiation component 3610. Such light sources may include characteristics indicative of ultraviolet-C (UV-C) light, otherwise referred to as shortwave ultraviolet light, such to disrupt the ability of pathogens and other materials to replicate after exposure to the emitted ultraviolet light. Additional germicidal light sources will be appreciated by those of skill in the art after having the benefit of this disclosure.

The nose plug component will now be discussed in greater detail. FIGS. 1-1-4, 6-13, 15-20, and 22-28 highlight examples of the nose plug component, which may also be shown in other figures. The nose plug component 1710, 2710, 3710, 4710 may be operatively attached to the housing 1210, 2210, 3210, 4210 to be removably inserted into a nostril such to form an at least partial seal with a nasal cavity. The nose plug component 1710, 2710, 3710, 4710 may be constructed of a durable material to minimize potential damage when they are being handled, which material being substantially nontoxic to humans, for example silicon or graphene.

The nose plug component 1710, 2710, 3710, 4710 may be operatively installed to at least part of the housing 1210, 2210, 3210, 4210 provided by an air purification apparatus enabled by this disclosure. The nose plug component 1710, 2710, 3710, 4710 may include a nose plug 1720, 2720, 3720 having a nose plug base end 1722, 2722, 3722, 4722 and a nose plug nostril end 1724, 2724, 3724, 4724. The nose plug 1720, 2720, 3720 may have a circumference and a diameter of a size such that, upon insertion into a nostril, the device may remain in place in the nostril anchored by friction. In one embodiment, the nose plug 1720, 2720, 3720 may have a diameter of between approximately 8 millimeters and approximately 14 millimeters and a length between approximately 15 millimeters and approximately 25 millimeters, without limitation.

The nose plug 1720, 2720, 3720 may include a nose plug aperture 1726, 2726, 3726, 4726 near the nose plug nostril end 1724, 2724, 3724, 4724. The nose plug 1720, 2720, 3720 may additionally include a nose plug protruding lip 1728, 2728, 3728 near the nose plug base end 1722, 2722, 3722, 4722. Additional features may be provided by the nose plug that would be appreciated by those of skill in the art after having the benefit of this disclosure.

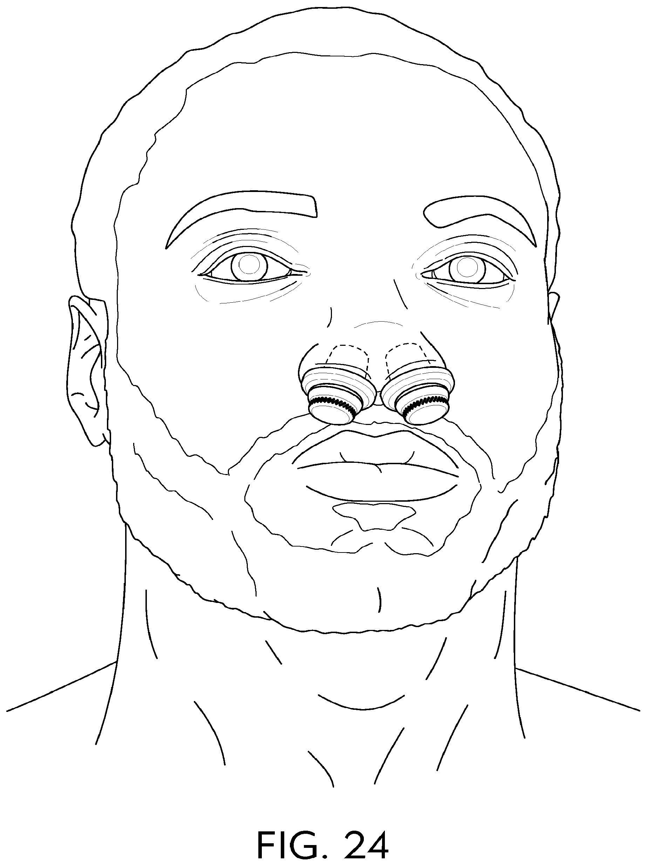

In one embodiment, provided without limitation, the nose plug 1720, 2720, 3720 may be constructed using an at least partially flexible material, for example silicone, such that it may be inserted into the nostril of a user with minimal or negligible irritation. Additionally, by using a flexible material, the nose plug may provide an at least partial seal within the nostril such as to provide access to the nasal cavity of a user upon operation. The nose plug 1720, 2720, 3720 may be configured such that the nose plug may be inserted at least partially into the nostril providing a pathway from the interior space provided by the housing 1210, 2210, 3210, 4210 through the nose plug 1720, 2720, 3720 and into the nasal cavity. Accordingly, when in use, such nose plug components 1710, 2710, 3710, 4710 may extend into a nostril to a depth of between approximately 15 millimeters and approximately 25 millimeters, without limitation, as illustrated in FIG. 24.

The nose plug 1720, 2720, 3720 may include a nose plug aperture 1726, 2726, 3726, 4726 that runs substantially throughout the interior of the nose plug. The nose plug aperture 1726, 2726, 3726, 4726 may extend substantially from the nose plug base end 1722, 2722, 3722, 4722 through the nose plug 1720, 2720, 3720 to the nose plug nostril end 1724, 2724, 3724, 4724. Air and/or gas may be moved through the nose plug aperture 1726, 2726, 3726, 4726 actively by operation of the fan component 1410, 3410 discussed above, passively through respiration by a user, via pressure from gas source, and/or otherwise as would be appreciated by those of skill in the art. In some embodiments, the nose plug aperture 1726, 2726, 3726, 4726 may include a mesh material capable of serving as an additional layer of filtration for any remaining undesired particulate matter that has not already been filtered out when air is moving through an air purification apparatus enabled by the present disclosure.

The nose plug component 1710, 2710, 3710, 4710 may include a nose plug protruding lip 1728, 2728, 3728, which may advantageously prevent the nose plug from being inserted undesirably deep within the nasal cavity of a user. The nose plug protruding lip 1728, 2728, 3728 may be substantially contiguous with the nose plug 1720, 2720, 3720 and may be constructed using the same materials as the nose plug. The nose plug protruding lip 1728, 2728, 3728 may advantageously provide a stopping mechanism, thus providing physical resistance from over-insertion of the nose plug 1720, 2720, 3720. In some embodiments, the nose plug protruding lip may have increased rigidity over the portion of the nose plug to be inserted into the nostril such to increase its stopping capability.

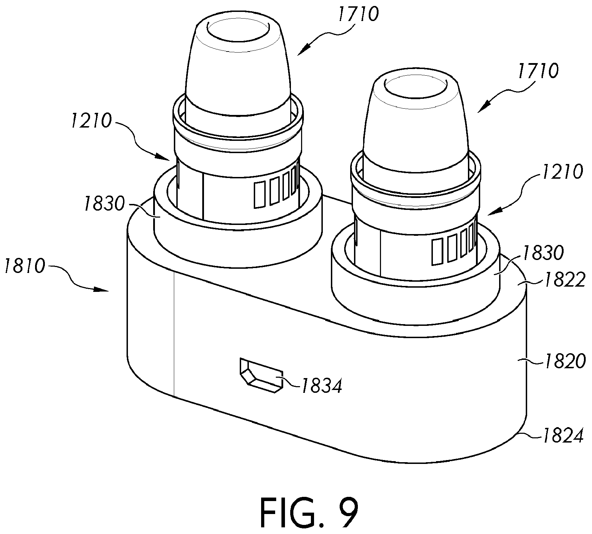

The case will now be discussed in greater detail. FIGS. 9-10 highlight examples of the case, which may also be shown in other figures. In some embodiments, a case 1810 may be provided to receive, store, charge, and otherwise interact with an air purification apparatus such as one enabled by this disclosure. For example, a case 1810 may be configured to receive an air purification apparatus when not in use such as when not being inserted into the nasal cavity of a user.

In one embodiment, the case 1810 may include a case enclosure 1820 which may extend from a case enclosure bottom end 1824 to a case enclosure top end 1822. The case enclosure 1820 may include case receiving slots 1830 at its case enclosure top end 1822 to receive and/or substantially secure the housing 1210 of an air purification apparatus enabled by this disclosure. The case enclosure receiving slot 1830 may include a case receiving slot aperture 1832 through which the housing 1210 may be inserted. The case enclosure receiving slot 1830 may include a protruding guideway which may interact with a depressed guideway included by the housing 1210 to assist with orienting the housing 1210 properly upon insertion into the case receiving slot 1830.

The case 1810 may include case power electronics 1840 to assist with charging an air purification apparatus enabled by this disclosure when inserted into the case 1810. For example, the case 1810 may include case power electronics 1840 capable of receiving external electrical power from an external power source via a case charging port 1842, which may be accessible via a case charging port access point 1834. The case power electronics 1840 may condition and/or otherwise affect the external power into a charging power capable of being received by the electrical contacts 1370 provided by the power component 1310 of the air purification apparatus enabled by the disclosure. For example, upon insertion of the housing 1210 of the air purification apparatus into the case receiving slot 1830, electrical contacts 1370 provided by the air purification apparatus may come into operative connection with electrical contacts 1844 provided by the case 1810. Once contact is established, charging power may be provided by the case 1810 to the battery 1340 of the air purification apparatus. In some embodiments, the electrical charging circuitry of the air purification apparatus may interact with power received by the case 1810 and condition such power such that it is storable in the battery 1340 included by the power component 1310. In other embodiments, power may be delivered by the case 1810 such that it may be applied directly to the battery 1340 and thus eliminate the need for separate electrical charging circuitry within the air purification apparatus itself.

The case 1810 may include a case charging port 1842, which may be provided as a USB port and/or other port capable of carrying electrical power, such as discussed above in the context of the power component 1310. In some embodiments, the case charging port 1842 may enable transmission of data received from the air purification apparatus located within the case receiving slot 1830. The case 1810 may operate as a pass-through device providing access between the case charging port 1842 provided by the case 1810 and the components held within the interior space of the air purification apparatus. In other embodiments, active electronics may be included within the case 1810 that can at least partially condition the power, facilitate the transmission of data, and/or otherwise assist with the operation of the air purification apparatus received by the case receiving slot 1830.

The case 1810 may include a case bottom panel 1826 located at the case enclosure bottom end 1824. The case bottom panel 1826 may be removably installed to the case 1810, which may provide access to an interior volume of the case upon removal from the case. One or more case feet 1828 may optionally be provided to improve the stability of the case 1810 when located on a surface and/or reduce the likelihood of damage to a surface on which the case may be located.

The monitoring component will now be discussed in greater detail. In one embodiment, a monitoring component may be provided that includes a sensor to detect a condition of the air included by an environmental space adjacent to the sensor. The condition may be reported via a communicably connected display device. The monitoring component may include one or more sensor components that may assist with monitoring aspects of user health, air quality, operational efficacy, and other metrics that would be understood by those of skill in the art. The monitoring component may additionally include technology capable of detecting presence or absence of potentially hazardous viruses, bacteria, and/or other agents in a user.

The sensors included by the monitoring component may detect one or more environmental conditions in the vicinity of a user of the devices. The environmental conditions may include, without limitation, presence or absence of airborne viruses, bacteria, carcinogens, and pollutants, as well as temperature, precipitation, and humidity. The sensor components may be communicatively and operatively connected to a display component capable of displaying data concerning the environmental conditions.

In another embodiment, the monitoring component may be used to indicate the likelihood of impurities, such as microorganisms, being present in an environmental space near the monitoring component. In some embodiments, the monitoring component may be located in the housing, and, thus, may be used to indicate the likelihood of microorganisms present in an environmental space accessible via the housing 4210. More particularly, the monitoring component may be able to detect the presence of microorganisms that enter the apparatus but have not yet been filtered. In other embodiments, the monitoring component may be located in the nose plug component. In these embodiments, the monitoring component may be used to indicate the likelihood of microorganisms present in an environmental space accessible via the nose plug component 4710. More particularly, the monitoring component may be able to detect the presence of microorganisms that have been exhaled by the user. Those skilled in the art, however, will appreciate the various locations of the monitoring component as well as the multiple purposes of the monitoring component after having the benefit of this disclosure.

In the same or other embodiments, the monitoring component may operate using a mechanical or chemical device, as will be appreciated by those of skill in the art. In these embodiments, the monitoring component may be used with or without electronics. In most embodiments, the monitoring component may provide evidence of there being a likelihood of impurities within an environmental space. Additionally, the monitoring component may test for various microbials and/or microorganisms. Some examples of devices that may be used in the monitoring component may include, but are not limited to, reactive test strips, reagents, stains, and disks. Those of skill in the art will appreciate the various other devices that may be used to test for impurities in an environmental space.

The monitoring component may operate using a computerized device including a processor capable of reading from memory and operating instructions stored in the memory, as will be appreciated by those of skill in the art. Alternatively, the monitoring component may assist with the analysis and presentation of information performed by an external computerized device associated with operation of the monitoring component, without limitation.

In operation, an air purification apparatus enabled by this disclosure may be used for purifying and/or reducing undesirable materials from air intended to enter a nasal cavity. Those of skill in the art will appreciate methods of use enabled by the above disclosure. Skilled artisans will appreciate additional methods within the scope and spirit of the disclosure for performing the operations provided by the examples herein after having the benefit of this disclosure. Such additional methods are intended to be included by this disclosure.

While various aspects have been described in the above disclosure, the description of this disclosure is intended to illustrate and not limit the scope of the invention. The invention is defined by the scope of the appended claims and not the illustrations and examples provided in the above disclosure. Skilled artisans will appreciate additional aspects of the invention, which may be realized in alternative embodiments, after having the benefit of the above disclosure. Other aspects, advantages, embodiments, and modifications are within the scope of the following claims. In addition to the claimed embodiments in the appended claims, the following is a list of additional examples which may serve as the basis for additional claims in this application or subsequent divisional applications:
Example 1: A gas delivery apparatus for nasal application comprising:
   a gas treatment component located within an interior space of a housing to at least partially purify gas for delivery to a nasal cavity comprising:
      a filtration component to remove impurities from the gas;
   a medicinal component to at least partially mix medicine with the gas received form the gas treatment component;
   a nose plug component operatively attached to a nose end of the housing to be removably inserted into a nostril such to form an at least partial seal with the nostril such that the gas being at least partially mixed with the medicine received from the medicinal component is directed to the nasal cavity through the nose plug component.
Example 2: The apparatus of example 1, wherein the impurities comprise microorganisms.
Example 3: The apparatus of example 1, wherein the impurities comprise particles having a size of more than approximately 0.3 microns.
Example 4: The apparatus of example 1, further comprising:
   a fan component to actively pass the gas through the filtration component.
Example 5: The apparatus of example 4, further comprising:
   a power component located substantially within the interior space to provide electrical power to the fan component.
Example 6: The apparatus of example 1, further comprising:
   an aroma component to receive the gas that has been at least partially purified to be mixed with an aroma released by an aromatic material housed by the aroma component; and
   wherein the aroma is at least partially mixed with the gas to induce a psychological response.
Example 7: The apparatus of example 1, further comprising:
   a cap removably attached to a cap end of the housing to receive the gas delivered from a gas source via a delivery line, the cap end being distal to the nose end.
Example 8: The apparatus of example 7, wherein the delivery line is received by the cap having a filtration diameter that is larger than a delivery line diameter to increase a filtration efficacy of the filtration component.
Example 9: The apparatus of example 8, wherein the cap comprises a cannula to receive the delivery line through which the gas flows from the gas source, wherein the delivery line diameter is larger than a cannula diameter of the cannula.
Example 10: The apparatus of example 7, wherein the gas source is a continuous positive airway pressure machine.
Example 11: The apparatus of example 7, wherein the gas source is a pressurized tank.
Example 12: The apparatus of example 7, wherein the housing has a cap receiving feature that is at least partially flexible to allow for installation and removal of the cap to the housing.
Example 13: The apparatus of example 1, wherein the filtration component comprises a high efficiency particulate absorbing (HEPA) filter that is replaceable.
Example 14: The apparatus of example 1, further comprising:
   a monitoring component that indicates a likelihood of the impurities being present in an environmental space accessible via the nose plug component.
Example 15: A gas delivery apparatus for nasal application comprising:
   a gas treatment component located within an interior space of a housing to at least partially purify a gas for delivery to a nasal cavity comprising:
      a high efficiency particulate absorbing (HEPA) filter to remove impurities from the gas;
   an aroma component having an aromatic material to mix the gas with an aroma emitted by the aromatic material to stimulate a psychological response;
   a nose plug component operatively attached to a nose end of the housing to be removably inserted into a nostril such to form an at least partial seal with the nostril such that the gas received from the aroma component is directed to the nasal cavity through the nose plug component to deliver the gas mixed with the aroma; and
   a cannula of a cap removably attached to a cap end of the housing to receive the gas delivered from a gas source via a delivery line to be directed to the gas treatment component, the cap end being distal to the nose end;
   wherein the delivery line having a delivery line diameter is selectively installed to the cannula such that the gas from the delivery line is received through a gas receiving aperture of the cannula.
Example 16: The apparatus of example 15, further comprising:
   a medicinal component to at least partially mix medicine with the gas received from the gas treatment component.
Example 17: The apparatus of example 15, wherein the impurities comprise particles having a size of more than approximately 0.3 microns.
Example 18: The apparatus of example 15, wherein a cap receiving feature of the housing is at least partially flexible to allow for installation and removal of the cap to the housing.
Example 19: The apparatus of example 15, further comprising an auxiliary aperture on the housing.
Example 20: The apparatus of example 15, wherein the delivery line is received by the cap having a filtration diameter that is larger than a delivery line diameter to increase a filtration efficacy of the high efficiency particulate absorbing (HEPA) filter.

## Claims

1. A gas purification apparatus for nasal application comprising:
a housing to substantially enclose an interior space;
a gas treatment component located within the interior space to at least partially purify gas for delivery to a nasal cavity comprising:
a filtration component to remove impurities from the gas;
a nose plug component operatively attached to a nose end of the housing to be removably inserted into a nostril such to form an at least partial seal with the nasal cavity such that the gas passed through the filtration component is directed to the nasal cavity through the nose plug component; and
a cap removably attached to a cap end of the housing to receive the gas delivered from a gas source via a delivery line, the cap end being distal to the nose end.

2. The apparatus of claim 1, the delivery line having a delivery line diameter that is selectively installed to the cap such that the gas from the delivery line is received through a gas receiving aperture of the cap.

3. The apparatus of claim 2, wherein the delivery line is received by the cap having a filtration diameter that is larger than the delivery line diameter to increase a filtration efficacy of the filtration component.

4. The apparatus of claim 2, wherein the cap comprises a cannula to receive the delivery line through which the gas flows, wherein the delivery line diameter is larger than a cannula diameter of the cannula.

5. The apparatus of claim 1, wherein the impurities comprise microorganisms.

6. The apparatus of claim 1, wherein the gas source is a continuous positive airway pressure machine.

7. The apparatus of claim 1, wherein the gas source is a pressurized tank.

8. The apparatus of claim 1, wherein the gas treatment component further comprises:
a fan component to actively pass the gas through the filtration component, and
a power component located substantially within the interior space to provide electrical power to the gas treatment component.

9. The apparatus of claim 1, wherein the gas treatment component further comprises:
an aroma component comprising an aroma reservoir to reversibly hold an aromatic material having an aroma; and
wherein the aroma is at least partially transferred from the aromatic material into the gas.

10. The apparatus of claim 1, wherein the filtration component is replaceable and comprises a high efficiency particulate absorbing (HEPA) filter.

11. The apparatus of claim 1, further comprising:
a monitoring component that indicates a likelihood of microorganisms being present in an environmental space accessible via the nose plug component.

12. A gas purification apparatus for nasal application comprising:
a housing to substantially enclose an interior space;
a gas treatment component located within the interior space to at least partially purify and at least partially remove moisture from gas for delivery to a nasal cavity comprising:
a high efficiency particulate absorbing (HEPA) filter to remove impurities from the gas;
a nose plug component operatively attached to a nose end of the housing to be removably inserted into a nostril such to form an at least partial seal with the nasal cavity such that the gas passed through the filtration component is directed to the nasal cavity through the nose plug component; and
a cannula of a cap removably attached to a cap end of the housing to receive the gas delivered from a gas source via a delivery line, the cap end being distal to the nose end;
wherein the delivery line having a delivery line diameter is selectively installed to the cannula such that the gas from the delivery line is received through a gas receiving aperture of the cannula, and
wherein the delivery line is received by the cap having a filtration diameter that is larger than the delivery line diameter to increase a filtration efficacy of the high efficiency particulate absorbing (HEPA) filter.

13. The apparatus of claim 1 or 12, wherein the gas treatment component further comprises:
a medicinal component comprising a medicinal reservoir to reversibly hold a medicine; and
wherein the medicine is at least partially transferred from the medicinal component into the gas.

14. The apparatus of claim 12, further comprising:
a monitoring component that indicates a likelihood of microorganisms being present in an environmental space accessible via the housing.

15. The apparatus of claim 1 or 12, wherein the housing has a cap receiving feature that is at least partially flexible to allow for installation and removal of the cap to the housing.

16. The apparatus of claim 1 or 12, further comprising an auxiliary aperture on the housing.

17. The apparatus of claim 16, further comprising a drying agent to at least partially remove moisture from within the interior space, and
wherein at least part of the moisture vents through the auxiliary aperture.

18. A gas purification apparatus for nasal application comprising:
a housing to substantially enclose an interior space;
a gas treatment component located within the interior space to at least partially purify gas for delivery to a nasal cavity comprising:
a filtration component to remove impurities from the gas; and
a nose plug component operatively attached to a nose end of the housing to be removably inserted into a nostril such to form an at least partial seal with the nasal cavity such that the gas passed through the filtration component is directed to the nasal cavity through the nose plug component.
